# EUROPEAN PATENT SPECIFICATION

(11) **EP 2 376 648 B1**
(45) Date of publication and mention of the grant of the patent: **14.09.2016**
(21) Application number: 09771304.4
(22) Date of filing: 08.12.2009
(51) Int. Cl.: C12Q 1/68

(54) **EPIGENETIC MARKERS FOR THE IDENTIFICATION OF CD3 POSITIVE T-LYMPHOCYTES**
EPIGENETISCHE MARKER ZUR IDENTIFIKATION VON CD3+ T-LYMPHOZYTEN
MARQUEURS ÉPIGÉNÉTIQUES POUR L'IDENTIFICATION DES LYMPHOCYTES T CD3+

(30) Priority: 16.12.2008 EP 08021838
(43) Date of publication of application: 19.10.2011
(73) Proprietor: Epiontis GmbH, 12489 Berlin (DE)
(72) Inventor: OLEK, Sven, 12207 Berlin (DE); TÜRBACHOVA, Ivana, 12207 Berlin (DE)
(74) Representative: Krauss, Jan
(86) International application number: PCT/EP2009/008764
(87) International publication number: WO 2010/069499

(56) References cited:
- FLANAGAN B F ET AL: "DNASE HYPERSENSITIVITY AND METHYLATION OF THE HUMAN CD3G AND D GENES DURING T-CELL DEVELOPMENT" IMMUNOGENETICS, SPRINGER VERLAG, BERLIN, DE, vol. 31, no. 1, 1 January 1990 (1990-01-01), pages 13-20, XP009113698 ISSN: 0093-7711
- CLEVERS H ET AL: "AN ENHANCER LOCATED IN A CPG-ISLAND 3' TO THE TCR-CD3-EPSILON GENE CONFERS T LYMPHOCYTE-SPECIFICITY TO ITS PROMOTER" EMBO (EUROPEAN MOLECULAR BIOLOGY ORGANIZATION) JOURNAL, vol. 8, no. 9, 1989, pages 2527-2536, XP002519563 ISSN: 0261-4189
- TUTT LANDOLFI M M ET AL: "Specific demethylation of the CD4 gene during CD4 T lymphocyte differentiation." MOLECULAR IMMUNOLOGY JAN 1997, vol. 34, no. 1, January 1997 (1997-01), pages 53-61, XP002519564 ISSN: 0161-5890
- LUSSO P ET AL: "Induction of CD4 and susceptibility to HIV-1 infection in human CD8<+> T lymphocytes by human herpesvirus 6" NATURE 1991 GB, vol. 349, no. 6309, 1991, pages 533-535, XP002519565 ISSN: 0028-0836
- GEORGOPOULOS K ET AL: "A T cell-specific enhancer is located in a DNase I-hypersensitive area at the 3' end of the CD3-delta gene." THE EMBO JOURNAL AUG 1988, vol. 7, no. 8, August 1988 (1988-08), pages 2401-2407, XP002519566 ISSN: 0261-4189

## Description

The present invention relates to a method, in particular an *in vitro* method, for identifying CD3⁺ CD4⁺, and/or CD3⁺ CD8⁺ naive and/or memory T-lymphocytes in a sample derived from a mammal comprising T cells, comprising a) analysing the methylation status of at least one CpG position in an amplicon comprising amplification with at least one primer pair suitably designed based on oligomers according to any of SEQ ID NO: 2 to 5, and b) identifying CD3⁺ CD4⁺, and/or CD3⁺ CD8⁺ naive and/or memory T-lymphocytes based on said methylation status, wherein a demethylation of at least one CpG position in said amplicon to at least 90% in said sample is indicative for a CD3⁺ CD4⁺, and/or CD3⁺ CD8⁺ naive and/or memoryT-lymphocyte cell. The method may further comprise analyzing the methylation status of at least one CpG position in the genes SLA2, CHRNA3, C16orf24, LCK, FASLG, CD7, SIT1, IL32, CXCR6, UBASH3A, GRAP2, ITGB7 and TXK which also allows the unambiguously identification of all CD3 positive T lymphocytes. For further unambiguous identification of all CD8 cells, also the equivalent analysis GNGT2, CRTAM, IL2RB and ZBTB32 can be employed. Among the CD3 positive T lymphocytes, these markers are capable to segregate between CD8 and CD4 positive cells. Equivalently, FLJ00060, FLJ38379, PPP6C, CD226, ZBTB7B and TNFAIP8 are capable of positively identifying CD4 expressing cells in whole blood and segregate between CD4 and CD8 positive CD3 positive cells.
The present invention provides a more robust means to quantitatively detect and measure particular subsets of the blood within any solid organs or any body fluid of a mammal.

Employing this method, the inventors provide for novel, not previously known means of determining, quantitating and routinely measuring T lymphocytes.

### Background of the invention

T-lymphocytes are a major component of the mammalian immune system. Both CD4 and CD8 T-cells are responsible for proper functioning of said immune system. Whereas CD8 T-cells mediate the cytotoxic immune defence, CD4 cells - the so called helper T-cells - assist both the humoral and the cell mediated immune defence. The heterodimeric T-cell antigen receptor (TCR) is bound to a monomorphic protein complex called CD3. CD3 consists of subelements CD3 γ, -δ, and -ε, and is expressed on all peripheral T-cells, but only on a subset of the thymocytes. The genes for CD3 γ, -δ, and -ε are encoded on neighbouring loci on chromosom 11 (11q23).

Role of CD3 in signal transduction - The function of the CD3-chains is both the formation and the transport of the full CD3 complex to the cell surface, as well as the signal transduction upon stimulation through the heterodimeric T-cell antigen receptor. The amino acid sequence of the cytoplasmatic part of CD3γ, -δ, -ε each contains a motif that becomes phosphorylated upon stimulation, and thus activated. This so called ITAM (immunoreceptor tyrosine-based activated motif) serves as docking point for different tyrosine kinases. Individuals with defective CD3γ or-ε-chains suffer from a severe clinical autoimmune deficiency.

Even though almost all cells in an individual contain the exact same complement of DNA code, higher organisms must impose and maintain different patterns of gene expression in the various types of tissue. Most gene regulation is transitory, depending on the current state of the cell and changes in external stimuli. Persistent regulation, on the other hand, is a primary role of epigenetics - heritable regulatory patterns that do not alter the basic genetic coding of the DNA. DNA methylation is the archetypical form of epigenetic regulation; it serves as the stable memory for cells and performs a crucial role in maintaining the long-term identity of various cell types.

The primary target of methylation is the two-nucleotide sequence Cytosine-Guanine (a 'CpG site'); within this context cytosine (C) can undergo a simple chemical modification to become 5-methyl-cytosine. In the human genome, the CG sequence is much rarer than expected, except in certain relatively dense clusters called 'CpG islands'. CpG islands are frequently associated with gene promoters, and it has been estimated that more than half of the human genes have CpG islands (Antequera and Bird, Proc Natl Acad Sci USA 90: 11995-9, 1993).

Aberrant methylation of DNA is frequently associated with the transformation from healthy to cancerous cells. Among the observed effects are genome-wide hypomethylation, increased methylation of tumour suppressor genes, and hypomethylation of many oncogenes (reviewed, for example, by Jones and Laird, Nature Genetics 21:163-167, 1999; Esteller, Oncogene 21:5427-5440, 2002; and Laird, Nature Reviews/Cancer 3:253-266, 2003). Methylation profiles have been recognized to be tumour specific (i.e., changes in the methylation pattern of particular genes or even individual CpGs are diagnostic of particular tumour types), and there is now an extensive collection of diagnostic markers for bladder, breast, colon, oesophagus, stomach, liver, lung, and prostate cancers (summarized, for example, by Laird, Nature Reviews/Cancer 3:253-266, 2003).

Flanagan et al. (in Flanagan BF, Wotton D, Tuck-Wah S, Owen MJ. DNase hypersensitivity and methylation of the human CD3G and D genes during T-cell development. Immunogenetics. 1990;31(1):13-20.) describe that the mouse and human CD3G and D genes are organized in opposite transcriptional orientation, their 5' ends being separated by about 1.6 kilobases (kb) of DNA. The molecular basis of the tissue-specific regulation of expression of the human CD3G and D genes were examined using DNase I hypersensitivity and CpG methylation analysis in the mouse. The authors try to define areas 3' to the D gene and within the intergenic region which contain regulatory elements that influence both CD3D and G expression and show that transcription from the CD3D and G genes may occur initially from a methylated promoter. Significantly, the 3' regulatory region was shown to adopt an open chromatin structure prior to lineage commitment and before CD3 transcription. The quite limited enzymatic analysis of Flanagan is based o a region in the mouse that has no homology to any region as found in the human. Furthermore, the paper does not identify the regions as analyzed as suitable for the identification of DC3 lymphocytes.

Clevers et al. (in Clevers H, Lonberg N, Dunlap S, Lacy E, Terhorst C. An enhancer located in a CpG-island 3' to the TCR/CD3-epsilon gene confers T lymphocyte-specificity to its promoter. EMBO J. 1989 Sep;8(9):2527-35.) describe that the gene encoding the CD3-epsilon chain of the T cell receptor (TCR/CD3) complex is uniquely transcribed in all T lymphocyte lineage cells. The human CD3-epsilon gene, when introduced into the mouse germ line, was expressed in correct tissue-specific fashion. The gene was then screened for T lymphocyte-specific cis-acting elements in transient chloramphenicol transferase assays. The promoter (-228 to +100) functioned irrespective of cell type. A 1225 bp enhancer with strict T cell-specificity was found in a DNase I hypersensitive site downstream of the last exon, 12 kb from the promoter. This site was present in T cells only. The CD3-epsilon enhancer did not display sequence similarity with the T cell-specific enhancer of CD3-delta, a related gene co-regulated with CD3-epsilon during intrathymic differentiation. The CD3-epsilon enhancer was unusual in that it constituted a CpG island, and was hypomethylated independent of tissue type. Two HTLV I-transformed T cell lines were identified in which the CD3-epsilon gene was not expressed, and in which the enhancer was inactive. In contrast to the preferred embodiment of the present invention, Clevers et al. analyze a remotely located enhancer region of 3' to the TCR/CD3-epsilon gene.

Hamerman et al. (in: Hamerman JA, Page ST, Pullen AM. Distinct methylation states of the CD8 beta gene in peripheral T cells and intraepithelial lymphocytes. J Immunol. 1997 Aug 1;159(3):1240-6) distinguish between CD4 and CD8 T-lymphocytes.

EP 1 213 360 describes a method of identifying a cell, tissue or nucleus, comprising collecting information on the methylation pattern of DNA isolated from the cell, tissue or nucleus and analyzing the resultant information.

WO 2004/050706 describes a sub-group of T-cells, and relates to characteristics of regulatory T-cells which define them as such. The application also describes the uses of such T-cells, compositions comprising them, and chemokines which recruit them in the modulation of an immune response.

Finally, EP 1 826 279 describes a method, in particular an *in vitro* method, for identifying FoxP3-positive regulatory T cells, preferably CD25⁺ CD4⁺ regulatory T cells of a mammal, comprising analyzing the methylation status of at least one CpG position in the gene foxp3 or an orthologous or paralogous gene thereof, and the use of DNA-methylation analysis of the gene of the transcription factor FoxP3 for a detection and quality assurance and control of regulatory T cells.

While the measurement and determination of CD4 and CD8 cells is generally easy and is usually achieved through analyzing the expression of said antigens on the cellular surface, clinically, it remains challenging to determine these cell types, since for the commonly used FACS analysis the cell samples need to be freshly isolated or immediately fixated in order to keep the cell entities intact. Thus, the detection of T lymphocytes, while desirous, is problematic, particularly for routine applications.

In view of the above, it is an object of the present invention to provide an improved and in particular robust method based on DNA methylation analysis as a superior tool in order to more conveniently and reliably identify T-lymphocytes.

The present invention solves the above object by providing a method for identifying CD3⁺ CD4⁺, and/or CD3⁺ CD8⁺ naive and/or memory T-lymphocytes in a sample derived from a mammal comprising T cells, comprising a) analysing the methylation status of at least one CpG position in an amplicon comprising amplification with at least one primer pair suitably designed based on oligomers according to any of SEQ ID NO: 2 to 5, and b) identifying CD3⁺ CD4⁺, and/or CD3⁺ CD8⁺ naive and/or memory T-lymphocytes based on said methylation status, wherein a demethylation of at least one CpG position in said amplicon to at least 90% in said sample is indicative for a CD3⁺ CD4⁺, and/or CD3⁺ CD8⁺ naive and/or memoryT-lymphocyte cell.

The present invention is based on the surprising finding of the inventors that the identification of CD3 gene as a specific epigenetic marker can greatly facilitate the clinical routine application of the analysis of the above markers. In contrast to FACS and mRNA measurements, the respective measurement(s) can be done independent of purification, storage and to quite some extend also to tissue quality.

In another preferred embodiment of the method according to the present invention, said at least one CpG position in said sample is demethylated to more than 91% and preferably more than 92% and most preferred more than 95%.

This concept is based on specific demethylation of the CD3 regions in CD3 positive T-lymphocytes. Using a simple and precise quantitative PCR method, the inventors show that CD3 demethylation represents a surrogate marker for T-lymphocyte counts in blood or tissues. The present inventors have thus identified particular regions within the CD3 gene, which are functionally involved in, or mandatory associated with, the existence of CD3 positive T-lymphocytes. In one preferred embodiment one very good region is either the promoter or the TLSDR with e.g. the nucleotide sequence according to SEQ ID No. 1 and others, containing many CpG motifs, which display a differential methylation status when cells expressing CD3 in either CD4⁺ or CD8⁺ cells compared with all other cells, not expressing CD3 if, for example, the bisulphite sequencing method is used.

The inventors could demonstrate that in CD3⁺ cells the CpG motifs are almost completely demethylated (i.e. to more than 90% and most preferred more than 95%), whereas the same motifs are completely methylated in all CD3- cells. The differential methylation of the CpG motifs within the aforementioned region correlates with CD3 expression. Thus, determination of the methylation status of the *CD3* locus could become a valuable tool to identify T-lymphocytes, such as will be required/or at least of some value for measuring T-lymphocytes in autoimmune diseases, transplant rejections, cancer, allergy, or just the T-lymphocytes related immune status in any envisionable context, when desired. The assays allows measurement if T-lymphocytes without purification or any staining procedures. It even reports in solid tumors or other solid tissues the number of cells demethylated in said region, thus showing the total amount of CD3 positive tumor infiltrating T-lymphocytes.

The inventors have shown that the potential for constitutive expression of CD3 in T-lymphocytes coincides with epigenetic, i.e., DNA methylation based regulation. DNA methylation is a biologically and chemically stable epigenetic modification, resulting in long-term gene expression changes. The inventors found demethylation at the human CD3 locus to be restricted to T-lymphocytes when tested against all major peripheral blood cell types and a selection of non-blood cells. These data indicated that epigenetic modifications in the CD3 locus serve as valuable marker for the identification of cells with the phenotype of T-lymphocyte, regardless of the expression of the specific delta or gamma sub-chains.

In another preferred aspect of the method according to the present invention the methylation status of at least one CpG position in the genes SLA2, CHRNA3, C16orf24, LCK, FASLG, CD7, SIT1, IL32, CXCR6, UBASH3A, GRAP2, ITGB7 and TXK is analysed in analogy to what is described herein for CD3. These genes thus also allow the unambiguous identification of all CD3 positive T lymphocytes. Thus, in a preferred embodiment of the method according to the present invention, said at least one CpG position is present in the 5' region upstream from the transcription start, promoter region, intron, and/or exon/intron border within the gene(s) SLA2, CHRNA3, C16orf24, LCK, FASLG, CD7, SIT1, IL32, CXCR6, UBASH3A, GRAP2, ITGB7 and TXK.

In order to further unambiguously identify all CD8 cells, the present invention in another preferred aspect thereof provides the equivalent analysis of the genes GNGT2, CRTAM, IL2RB and ZBTB32 among the CD3 positive T lymphocytes, as these markers are capable to segregate between CD8 and CD4 positive cells. This analysis is preferably performed simultaneously or subsequently to the analysis for the CD3 phenotype of the T-lymphocytes.

Equivalently, FLJ00060, FLJ38379, PPP6C, CD226, ZBTB7B and TNFAIP8 are capable of positively identifying CD4 expressing cells in whole blood and segregate between CD4 and CD8 positive CD3 positive cells.

Another preferred aspect of the method according to the present invention is directed at the use of DNA-methylation analysis of the genes CD3χ/δ/ε, or SLA2, CHRNA3, C16orf24, LCK, FASLG, CD7, SIT1, IL32, CXCR6, UBASH3A, GRAP2, ITGB7 and TXK or GNGT2, CRTAM, IL2RB and ZBTB32 or FLJ00060, FLJ38379, PPP6C, CD226, ZBTB7B and TNFAIP8 for the detection and quality assurance and control of T lymphocytes.

Said analysis of the methylation status comprises amplification with at least one primer of suitable primer pairs that can be suitably designed based on oligomers according to SEQ ID No. 2 to 5.

Preferably, the amplification involves a polymerase enzyme, a PCR or chemical amplification reaction, or other amplification methods as known to the person of skill as described below, e.g. in the context of MSP, HeavyMethyl, Scorpion, MS-SNUPE, MethylLight, bisulfite sequencing, methyl specific restriction assays. With the amplification, the amplicon of the TLSDR or any other region in the CD3 gene or any paralog or ortholog as described herein is produced that is a particularly preferred "tool" for performing the method(s) according to the present invention.

The person of skill will furthermore be able to select specific subsets of CpG positions in order to minimise the amount of sites to be analyzed. The positions are numerically counted from the 5'-end of the amplicon as generated and analysed. Preferred are combinations of 4, 5, 6, or 7 positions, which are producing enough information in order to be informative in the context of the present invention.

In order to analyze the methylation status of CpG positions, any known method to analyse DNA methylation can be used. In a preferred embodiment of the method according to the present invention, the analysis of the methylation status comprises a method selected from methylation specific enzymatic digests, bisulphite sequencing, analysis selected from promoter methylation, CpG island methylation, MSP, HeavyMethyl, MethyLight, Ms-SNuPE or other methods relying on a detection of amplified DNA. These methods are well known to the person of skill, and can be found in the respective literature.

In a preferred embodiment of the method according to the present invention, said method is suitable for routine application, for example on a DNA-chip. Based on the above information and the respective literature, the person of skill will be able to adjust the method as above to such settings.

In another preferred embodiment of the method according to the present invention, the identification comprises a distinction of said T-lymphocytes from all major peripheral blood cell types or non-blood cells, preferably, but not limited to, CD14, CD15, CD19, and CD56.

In yet another preferred embodiment of the method according to the present invention, the sample is selected from a mammalian body fluid, including human blood samples, or a tissue, organ or cell type blood sample, a sample of blood lymphocytes or a fraction thereof Preferably, said mammal is a mouse, rat, monkey or human. The samples can be suitably pooled, if required.

Another preferred aspect of the method according to the present invention then further comprises the step of concluding on the immune status of said mammal based on said T-lymphocytes as identified. The general lymphocyte population can be quantified and either be used as a benchmark to relatively quantify further detailed subpopulations such as the CD3CD4CD25 positive regulatory T cells or it can be used to finally detect this population to determine the overall immune activity status.

In yet another preferred embodiment of the methods according to the present invention, the mammal suffers from or is likely to suffer from autoimmune diseases, transplant rejections, cancer, and/or allergy.

Disclosed is an oligomer according to any of SEQ ID No. 2 to 5 or an amplicon as amplified by a primer pair based on SEQ ID No. 1 and designed as described above, in particular the amplicon No. 1405 (SEQ ID No. 6), amplicon No. 1406 (SEQ ID No. 7), and amplicon No. 1408 (SEQ ID No. 8).

Yet another preferred aspect of the present invention then relates to the use of a kit comprising materials for performing the method for identifying CD3⁺ CD4⁺, and/or CD3⁺ CD8⁺ naive and/or memory T-lymphocytes in a sample according to a method according the present invention as described herein, said kit comprising materials for performing a methylation analysis for CpG positions in an amplicon comprising amplification with at least one primer pair suitably designed based on oligomers according to any of SEQ ID NO: 2 to 5.

In summary, using the CD3 marker, the inventors very specifically identified (but not differentiated) both CD3/CD4 positive as well as CD3/CD8 positive T lymphocytes. Using the marker CD8beta, for example CD8 positive lymphocytes could then be distinguished from CD4 lymphocytes. This, when using a combination of the present marker(s) and the CD8beta marker, CD4 and CD8 cells can be specifically distinguished. This was not possible before the invention, since all CD4 looked identical to non-T lymphocytes.

The invention will now be further described based on the following examples and with reference to the accompanying figures and the sequence protocol, without being limited thereto. In the Figures and Sequences,
Figure 1 shows the differentially methylated gene region as found for CD3 g and d and are indicated by red lines as "blast hits".
Figure 2 shows the analysis of amplicon No. 1405 (SEQ ID No. 6), bisulfite strand 2, sequencing direction: forward. Relevant positions are indicated in bold.
Figure 3 shows the analysis of amplicon No. 1406, bisulfite strand 2, sequencing direction: reverse. Relevant positions are indicated in bold.
Figure 4 shows the analysis of amplicon No. 1406 (SEQ ID No. 7), bisulfite strand: 2, sequencing direction: forward. Relevant positions are indicated in bold.
Figure 5 shows the analysis of amplicon No. 1408 (SEQ ID No. 8), bisulfite strand: 2, sequencing direction: forward. Relevant positions are indicated in bold.

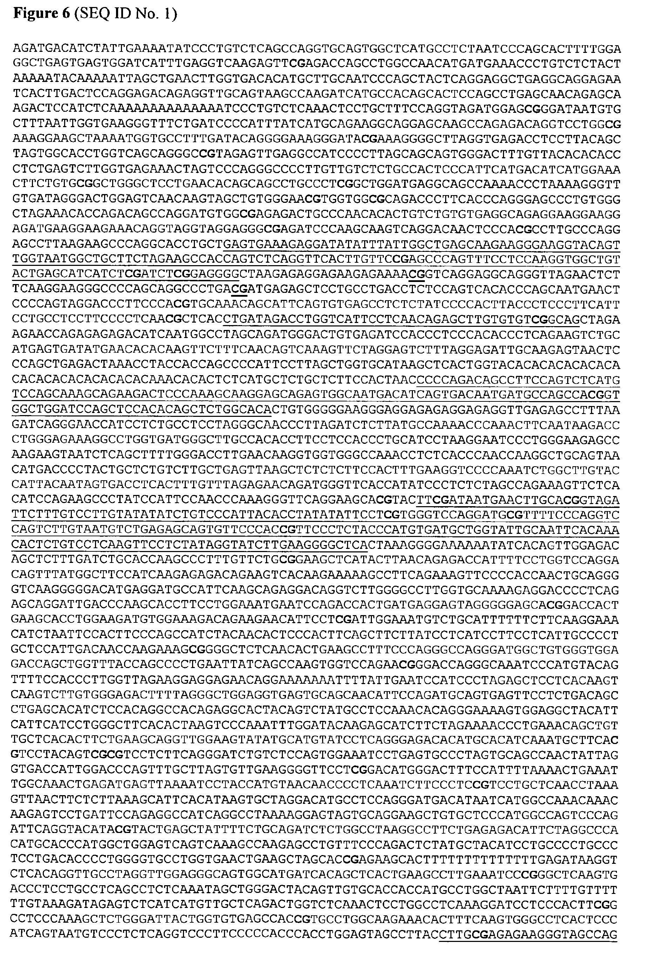

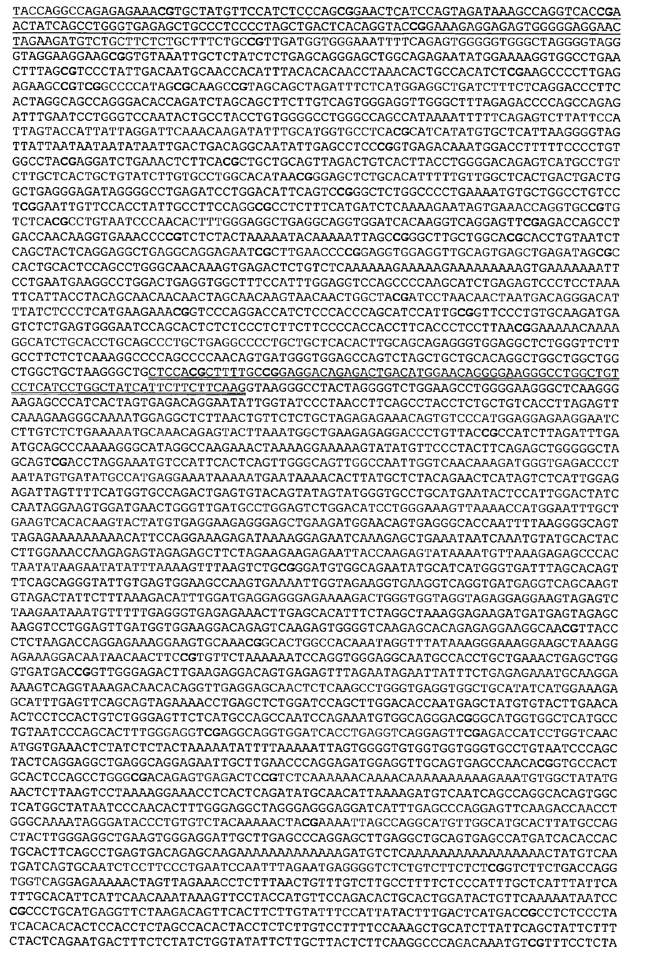

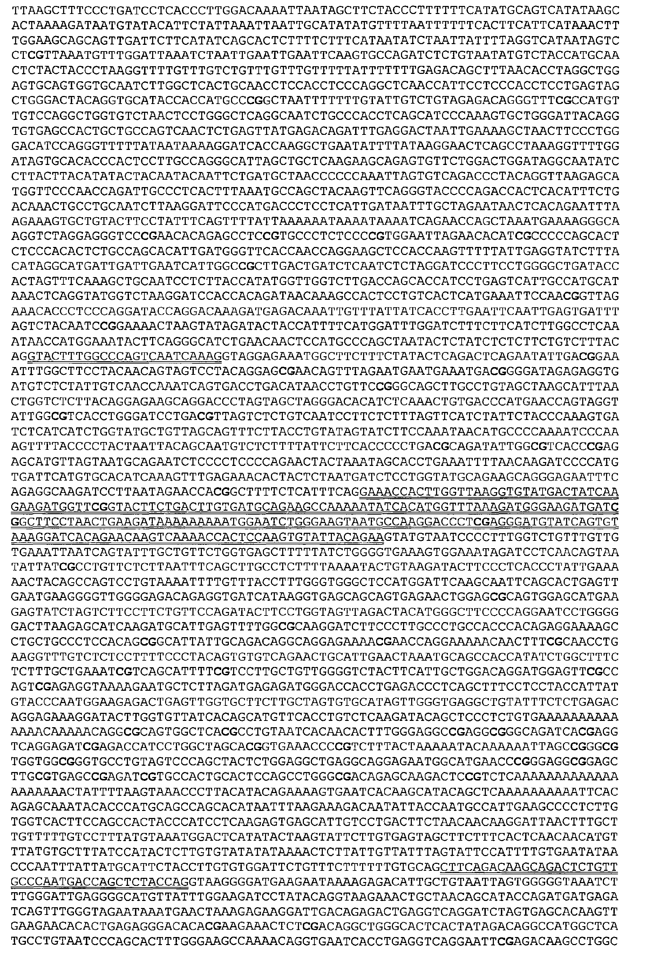

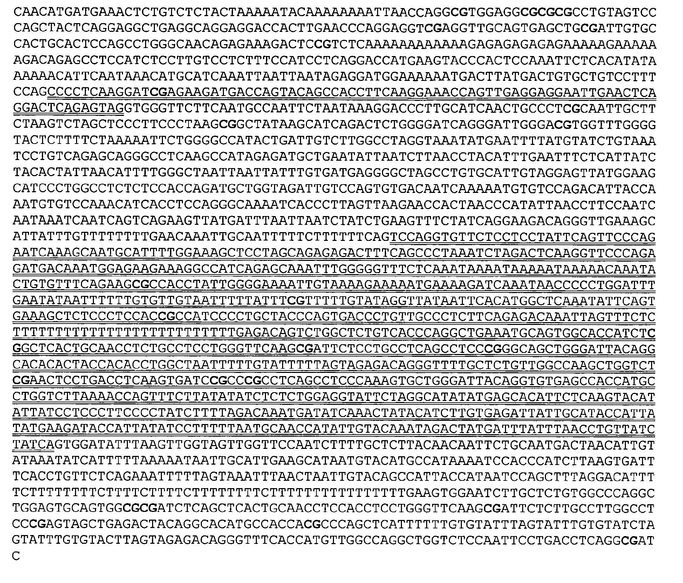

Figure 6 shows the regulatory regions of the γ,δ-T-cell receptor, including regions Nr. 1405 1406 und 1408 on chromosome 11 (NCBI36:11:117714000:117730500:1). Exon sequences are underlined for CD3 δ, and double underlined for CD3 γ. CGs are in bold.

SEQ ID No. 1 shows the sequence of the region as considered to have a specific methylation pattern in CD3 cells.

SEQ ID No. 2 to 5 and 9 show the sequences of oligonucleotides used in the Illumina Chip-Fragment assay for CD3γ and CD3δ and CD3ε.

SEQ ID No. 6 to 8 show the reference sequences of amplicon No. 1405 (SEQ ID No. 6), amplicon No. 1406 (SEQ ID No. 7), and amplicon No. 1408 (SEQ ID No. 8).

SEQ ID No. 10 to 38 show the sequences around the CpG positions as analyzed in other preferred T-lymphocyte markers of the present invention according to example 2.

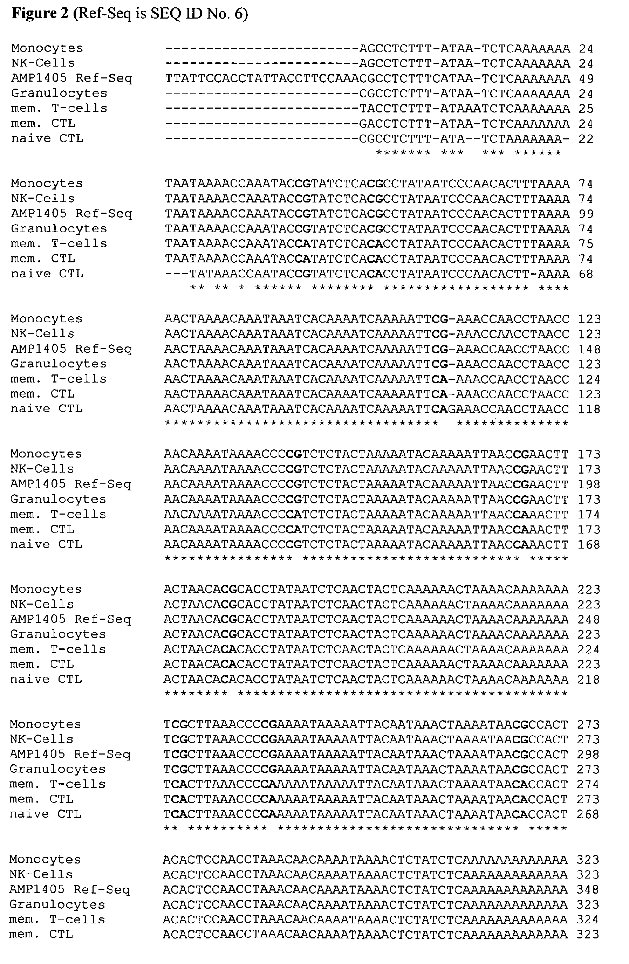

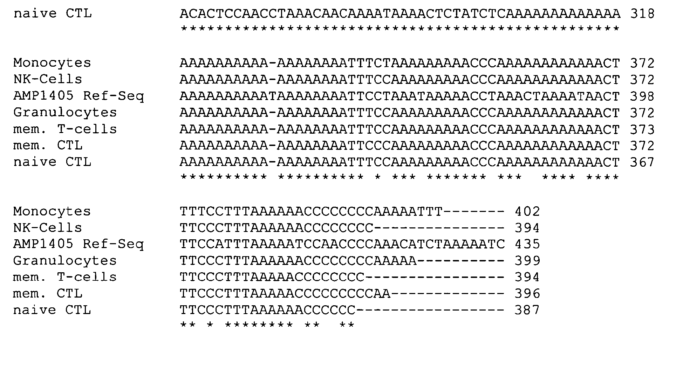

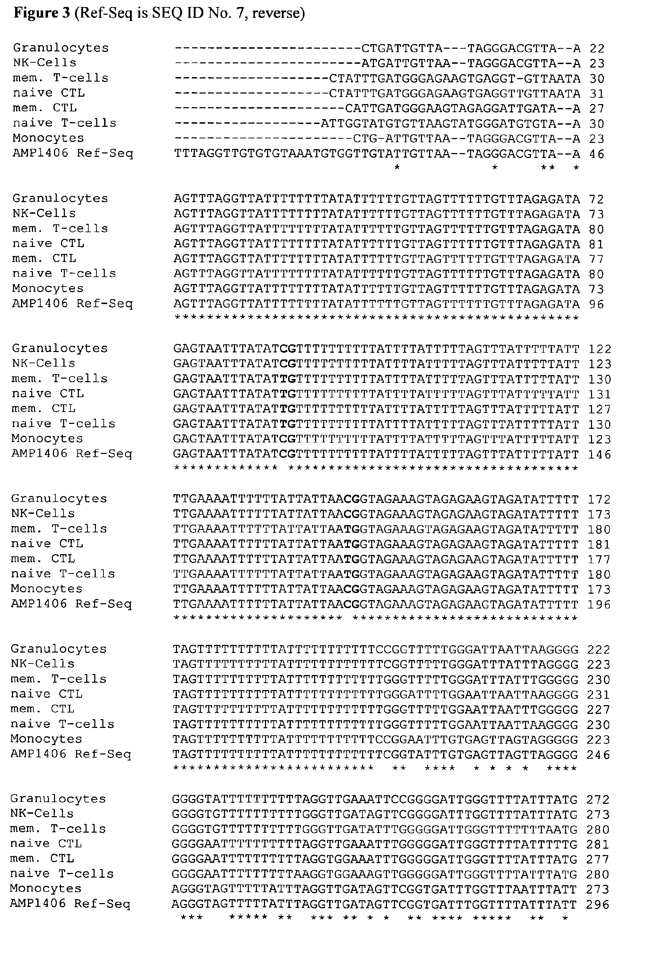

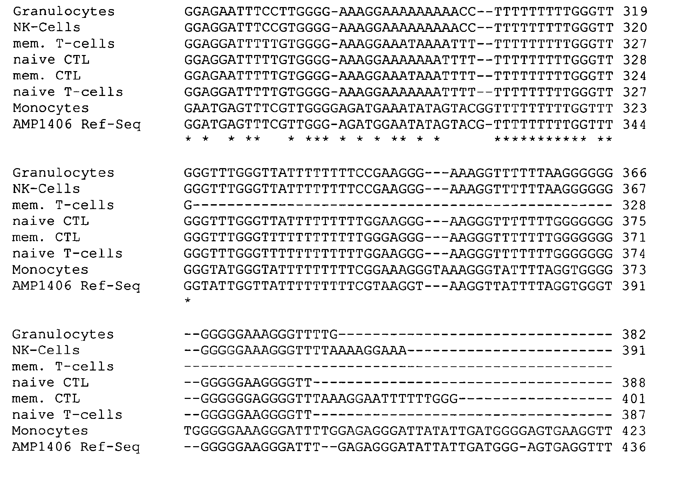

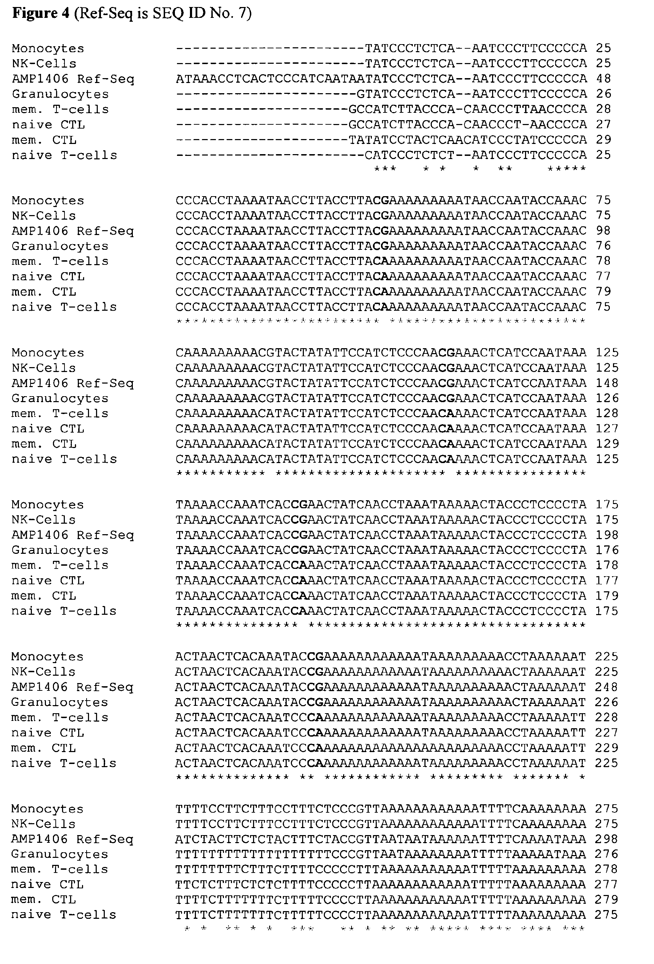

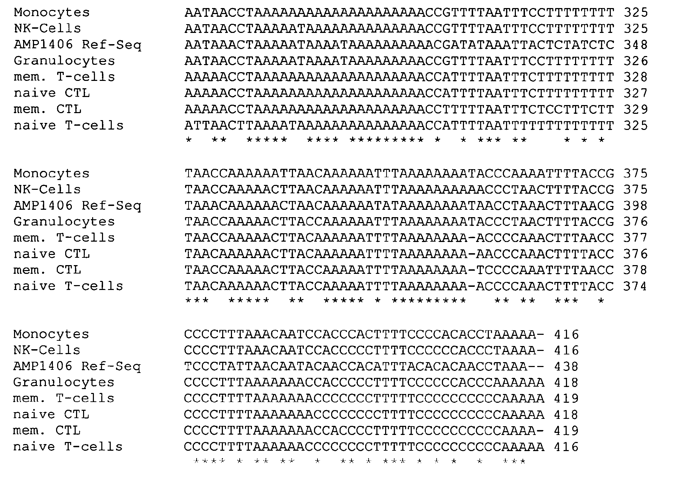

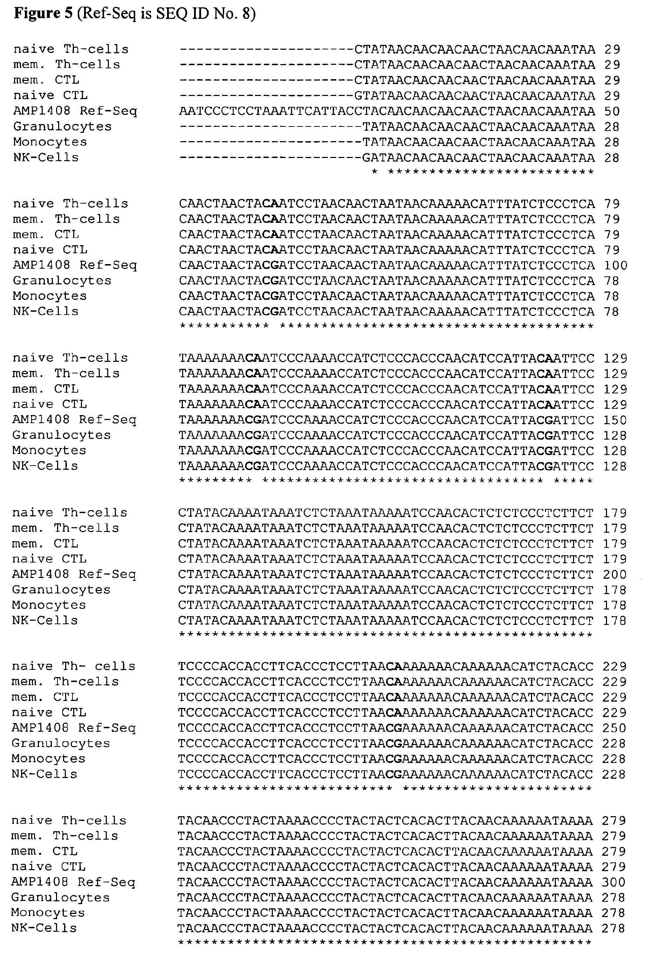

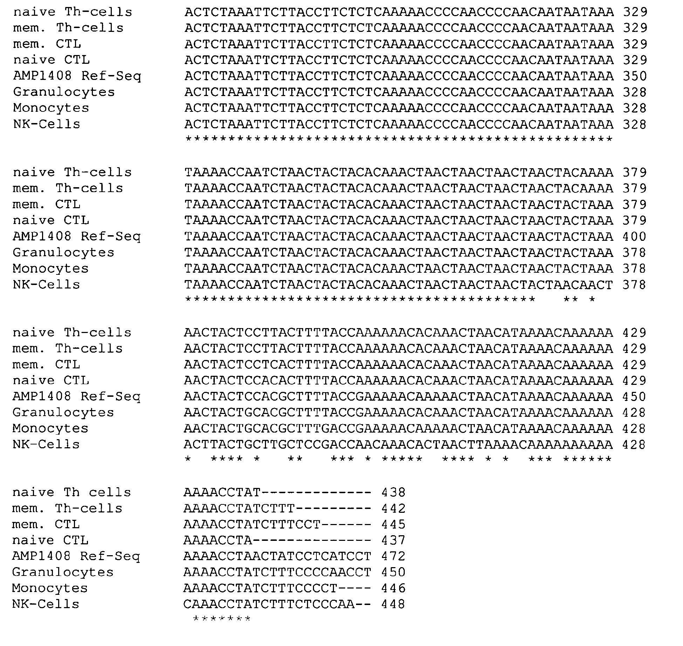

SEQ ID No. 39 to 65 show the sequences as depicted in the alignments of figures 2 to 5. If not stated otherwise, the sequences are ordered in the succession of their appearance in figures 2 to 5.

### EXAMPLES

### Example 1 - CD3-Analysis

The inventors have purified various blood subsets, including CD3/CD4, CD3/CD8 naive and memory T lymphocytes, CD56 natural killer cells, CD19 naive and memory B cells, CD14 monocytes and CD 15 granulocytes. DNA from the purified cells was bisulfite-treated and analysed at various CpG dinucleotide motifs. The inventors then compared the methylation status (finding C as for Cytosine that was methylated in the original sequence versus T for cytosine that was unmethylated in the original sequence).

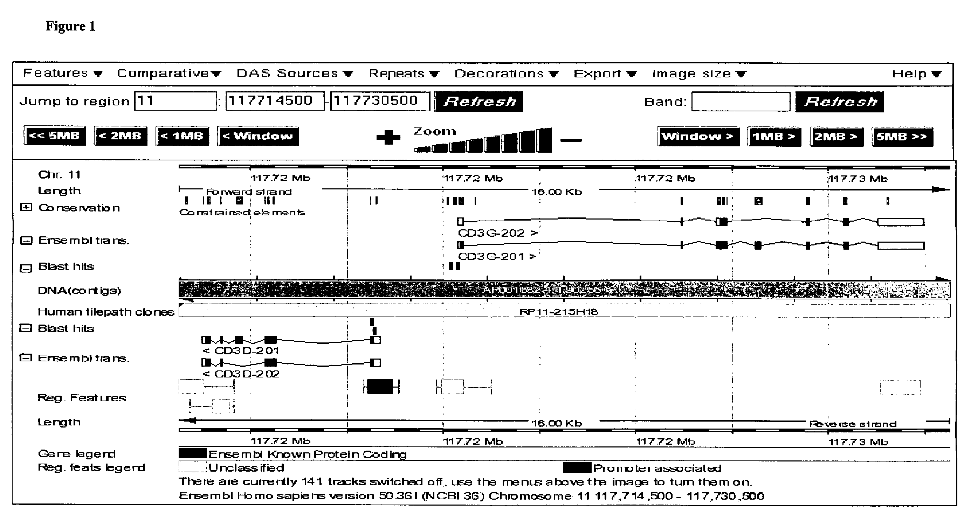

The data showed various CpG motifs and areas in the CD3 γ, δ and ε that were demethylated in all CD3CD4 and CD3CD8 cell types while methylated in all other blood cell types. The differentially methylated gene regions as found for CD3 γ, δ and ε are shown below in Figure 1 and are indicated in bold as "blast hits"

The data, as observed with Illumina Golden Gate technology, show that all CD4 and CD8 positive memory (0.06 and 0.06 respectively) and naive (0.03 and 0.06, respectively) T cells are subject to much lower methylation rates than all other tested cell types, including CD15 (0.92), CD 14 (0.90), CD 19 memory and naive (0.81 and 0.67, respectively), CD56 (0.86) positive blood cells as well as cells derived (0.78) from non-blood tissues. The experimental results are further depicted in the following table.

**Table 1: Methylation analysis results**

| **Gene** | **Chr.** | **S1** | **S2** | **S3** | **S4** | **S5** | **S6** | **S7** | **S8** | **S9** | **S10** | **S11** | **S12** |
|---|---|---|---|---|---|---|---|---|---|---|---|---|---|
| | | n=12; female | n=5; male | Pool; female | Pool; (5 male) | Pool; (5 male) | Pool; (5 male) | Pool; (5 male) | Pool; (5 male) | Pool; (5 male) | Pool; (5 male) | Pool; (5 male) | Pool; (5 male) |
| CD3D | 11 | 0.8 | 0.6 | 0.6 | 0.9 | 0.9 | 0.9 | 0.0 | 0.1 | 0.1 | 0.1 | 0.7 | 0.8 |
| | | 0.9 | 0.7 | 0.7 | 0.9 | 0.9 | 0.9 | 0.1 | 0.1 | 0.2 | 0.2 | 0.7 | 0.8 |
| CD3G | 11 | 0.7 | 0.6 | 0.7 | 0.9 | 0.9 | 0.8 | 0.1 | 0.1 | 0.1 | 0.1 | 0.8 | 0.9 |
| | | 0.4 | 0.6 | 0.5 | 0.7 | 0.8 | 0.7 | 0.1 | 0.1 | 0.1 | 0.1 | 0.3 | 0.6 |

| | | | | | | | | | | | | | |
|---|---|---|---|---|---|---|---|---|---|---|---|---|---|
| S1 = ovarian tissue, S2 = whole blood, S3 peripheral blood mononuclear cells, S4 = granulocytes, S5 = monocytes,S6 = NK cells, S7 = naïve T helper cells, S8 = memory T helper cells, S9 = naive cytotoxic T cells, S10 = memory cytotoxic T cells, S11 = naive B-cells, S12 = memory B-cells., Chr = chromosome | | | | | | | | | | | | | |

The data showing the high specificity were obtained using a Illumina Golden Gate technology, with the following genomic CpGs regions analysed

### CD3γ

cg15880738 (SEQ ID No. 2) (+1)**CG**GAAAAACAAAAGGCATCTGCACCTGCAGCCCTGCTGAGGCCCCTGCTG

### CD3δ

cg24841244 (SEQ ID No. 4) (-1) TGGAACATAGCA**CG**TTTCTCTCTGGCCTGGTACTGGCTACCCTTCTCT**CG**

### CD3ε

cg24612198 (Seq ID No. 9)
   AGTCATCTGTTTTGCTTTTTTTCCAGAAGTAGTAAGTCTGCTGGCCTCCG

Since the Illumina technology does only allow the analysis of a single CpG (or rather 2 CpGs per gene locus) the inventors verified the methylation properties using bisulfite sequencing. For that, the inventors bisulfite treated the samples using the Qiagen EpiTect kit, and sequenced the samples using an ABI 3100 prism sequencer. For data interpretation, the KB base calling software supplied by ABI was used.

While not providing entirely quantitative results calculating the percentile of methylated (i.e., CG signal on the plus strand sequence) versus unmethylated (i.e., TG on the plus strand sequence), the data were unambiguous for the purified cell types. All T-lymphocytes were overwhelmingly demethylated at all CG positions analysed, whereas all other analysed cell types were methylated at identical positions.

### Example 2 -Analysis of additional markers in analogy to CD3

In order to identify further suitable markers distingushing and monitoring T-lymphocytes, other markers in addition to CD3 have been identified and tested through methylation analysis. It was found that methylation in the CpG positions in the genes for SLA2, CHRNA3, C16orf24, LCK, FASLG, CD7, SIT1, IL32, CXCR6, UBASH3A, GRAP2, ITGB7 and TXK can also be used in the context of the present invention, as these markers are also able to identify CD3 positive T lymphocytes.

Furthermore, other markers have been identified that identify the subset of the CD8 and CD4 positive cells in the group of CD3 positive T lymphocytes. The genes for GNGT2, CRTAM, IL2RB and ZBTB32 have been found to segregate between CD8 and CD4 positive cells. Equivalently, FLJ00060, FLJ38379, PPP6C, CD226, ZBTB7B and TNFAIP8 are capable of positively identifying CD4 expressing cells in whole blood and segregate between CD4 and CD8 positive CD3 positive cells.

The following table 2 summarizes the Illumina-data as obtained for the above markers at selected CpG positions. It can be seen that several other markers can be used in order to selectively identify CD4+ (for CD3+ lymphocyte subset-identification), namely FLJ00060; FLJ38379; PPP6C; CD226; ZBTB7B and/or TNFAIP8, in order to selectively identify CD8+ (for CD3+ lymphocyte subset-identification), namely GNGT2; CRTAM; IL2RB and/or ZBTB32, and in order to selectively identify CD3+ lymphocytes, namely CD3D; CD3G, and/or CD3E, and/or SLA2, CHRNA3, C16orf24; LCK; FASLG; FASLG; CD7; SIT1; IL32; CXCR6; UBASH3A; GRAP2; ITGB7 and/or TXK, as shown with selected CpG sites as preferred examples. Based on the table, the person of skill will be able to extend the teaching regarding CD3 as herein to these markers and their CpG sites.

**Table 2: Illumina-data as obtained selected markers at CpG positions Chr. = chromosome**

| CD4+ marker CpG-ID | Gene Name | Ch r. | SEQ ID No. / Accession No 10/ | Ovar Tissue (mean, n=12) | Whole Blood (Pool) | PBMC (Promega) | BCST1 8 Granulocyte (CD15 +) | BCST1 9 Monocyte (CD14 +) | BCST2 0 NK (CD56 +) | BCST21 T naive (CD4+C D27 +CD45R A+) | BCST22 T mem (CD4+ CD27+ CD45RA -) | BCST23 CTL naive (CD8+C D27+ CD45RA +) | BCST24 CTL mem (CD8+C D27+ CD45RA -) | BCST2 5 B naive (CD19 +) | BCST2 6 B mem (CD19 +) | Mean Value Target Cell Type | Mean Value Rest | Delta Meth (Target-Rest) |
|---|---|---|---|---|---|---|---|---|---|---|---|---|---|---|---|---|---|---|
| cg03602 500 | FLJ000 60 | 19 | NM_0332 06.1 | 0.802 | 0.728 | 0.779 | 0.858 | 0.851 | 0.873 | 0.235 | 0.279 | 0.521 | 0.687 | 0.656 | 0.747 | 0.257 | 0.75 | -0.493 |
| cg16173 109 | FLJ383 79 | 2 | 11/ XR_00102 6.1 | 0.614 | 0.675 | 0.719 | 0.833 | 0.844 | 0.784 | 0.1141 | 0.113 | 0.341 | 0.383 | 0.418 | 0.385 | 0.113 | 0.599 | -0.486 |
| cg00620 024 | PPP6C | 9 | 12/ NM_0027 21.3 | 0.641 | 0.619 | 0.692 | 0.801 | 0.851 | 0.836 | 0.107 | 0.169 | 0.269 | 0.420 | 0.404 | 0.502 | 0.138 | 0.603 | -0.465 |
| cg13164 537 | CD226 | 18 | 13/ NM_0065 66.1 | 0.458 | 0.470 | 0.508 | 0.582 | 0.557 | 0.601 | 0.029 | 0.071 | 0.351 | 0.543 | 0.366 | 0.501 | 0.05 | 0.494 | -0.443 |
| cg01782 486 | ZBTB7 B | 1 | 14/ NM_0158 72.1 | 0.789 | 0.725 | 0.720 | 0.823 | 0.809 | 0.886 | 0.190 | 0.428 | 0.875 | 0.864 | 0.586 | 0.414 | 0.309 | 0.749 | -0.44 |
| eg07086 380 | TNFAI P8 | 5 | 15/ NM_0143 50.1 | 0.728 | 0.579 | 0.635 | 0.857 | 0.733 | 0.793 | 0.110 | 0.106 | 0.288 | 0.148 | 0.309 | 0.208 | 0.108 | 0.528 | -0.419 |

| CD8+ marker CpG-ID | Gene Name | Ch r | SEQ ID No. / Accession No | Ovar Tissue (mean, n=12) | Whole Blood (Pool) | PBMC (Pro-mega) | BCST1 8 Granulocyte (CD15 +) | BCST1 9 Monocyte (CD14 +) | BCST2 0 NK (CD56 +) | BCST21 T naive (CD4+C D27 +CD45R A+) | BCST22 T mem (CD4+ CD27+ CD45RA -) | BCST23 CTL naive (CD8+C D27+ CD45RA +) | BCST24 CTL mem (CD8+C D27+ CD45RA -) | BCST2 5 B naive (CD19 +) | BCST2 6 B mem (CD19 +) | Mean Value Target Cell Type | Mean Value Rest | Delta Meth (Target-Rest) |
|---|---|---|---|---|---|---|---|---|---|---|---|---|---|---|---|---|---|---|
| cg17839 611 | GNGT 2 | 17 | 16/ NM_0314 98.1 | 0.779 | 0.629 | 0.688 | 0.828 | 0.867 | 0.207 | 0.340 | 0.562 | 0.09 | 0.119 | 0.854 | 0.722 | 0.104 | 0.648 | -0.543 |
| cg22512 531 | CRTA M | 11 | 17/ NM_0196 04.2 | 0.651 | 0.634 | 0.709 | 0.835 | 0.735 | 0.5 | 0.363 | 0.630 | 0.079 | 0.082 | 0.194 | 0.336 | 0.08 | 0.559 | -0.478 |
| cg26757 673 | IL2RB | 22 | 18/ NM_0008 78.2 | 0.799 | 0.662 | 0.752 | 0.878 | 0.9 | 0.092 | 0.573 | 0.086 | 0.157 | 0.061 | 0.411 | 0.403 | 0.109 | 0.555 | -0.446 |
| cg08539 991 | ZBTB3 2 | 19 | 19/ NM_0143 83.1 | 0.823 | 0.736 | 0.791 | 0.912 | 0.926 | 0.674 | 0.563 | 0.153 | 0.303 | 0.085 | 0.648 | 0.140 | 0.194 | 0.637 | -0.442 |

| CD4+ / CD8+ marker CpG-ID | Gene Name | Ch r | SEQ ID No. / Accession No 20/ | Ovar Tissue (mean, n=12) | Whole Blood (Pool) | PBMC (Promega) | BCST1 8 Granulocyte (CD15 +) | BCST1 9 Monocyte (CD14 +) | BCST2 0 NK (CD56 +) | BCST21 T naive (CD4+C D27 +CD45R A+) | BCST22 T mem (CD4+ CD27+ CD45RA -) | BCST23 CTL naive (CD8+C D27+ CD45RA +) | BCST24 CTL mem (CD8+C D27+ CD45RA -) | BCST2 5 B naive (CD19 +) | BCST2 6 B mem (CD19 +) | Mean Value Target Cell Type | Mean Value Rest | Delta Meth (Target-Rest) |
|---|---|---|---|---|---|---|---|---|---|---|---|---|---|---|---|---|---|---|
| cg24841 244 | CD3D | 11 | NM_0007 32.3 | 0.783 | 0.582 | 0.644 | 0.917 | 0.904 | 0.857 | 0.025 | 0.057 | 0.057 | 0.058 | 0.673 | 0.810 | 0.049 | 0.771 | -0.721 |
| cg07728 874 | CD3D | | 21/ NM_0007 32.3 | 0.854 | 0.683 | 0.727 | 0.911 | 0.901 | 0.881 | 0.128 | 0.149 | 0.175 | 0.155 | 0.744 | 0.807 | 0.152 | 0.814 | -0.661 |
| cg15880 738 | CD3G | 11 | 22/ NM_0000 73.1 | 0.714 | 0.616 | 0.682 | 0.874 | 0.876 | 0.836 | 0.061 | 0.091 | 0.093 | 0.103 | 0.811 | 0.898 | 0.087 | 0.788 | -0.701 |
| cg07545 925 | CD3G | | 23/ NM_0000 73.1 | 0.370 | 0.552 | 0.545 | 0.737 | 0.771 | 0.739 | 0.133 | 0.149 | 0.132 | 0.131 | 0.303 | 0.601 | 0.132 | 0.49 | -0.358 |
| cg24612 198 | CD3E | 11 | 24/ NM_0007 33.2 | 0.679 | 0.485 | 0.563 | 0.794 | 0.793 | 0.698 | 0.064 | 0.036 | 0.093 | 0.051 | 0.206 | 0.279 | 0.061 | 0.562 | -0.501 |
| cg04759 756 | SLA2 | 20 | 25/ NM_0322 14.2 | 0.857 | 0.673 | 0.754 | 0.892 | 0.925 | 0.711 | 0.363 | 0.228 | 0.129 | 0.211 | 0.756 | 0.849 | 0.233 | 0.802 | -0.569 |
| cg22670 733 | CHRN A3 | 15 | 26/ NM_0007 43.2 | 0.792 | 0.733 | 0.721 | 0.911 | 0.888 | 0.8 | 0.122 | 0.180 | 0.092 | 0.315 | 0.456 | 0.668 | 0.177 | 0.746 | -0.568 |
| cg09830 866 | C16orf 24 | 16 | 27/ NM_0239 33.1 | 0.493 | 0.629 | 0.647 | 0.842 | 0.79 | 0.076 | 0.041 | 0.056 | 0.033 | 0.058 | 0.72 | 0.510 | 0.047 | 0.588 | -0.541 |
| cg17078 393 | LCK | 1 | 28/ NM_0053 56.2 | 0.807 | 0.575 | 0.666 | 0.920 | 0.884 | 0.26 | 0.05 | 0.037 | 0.036 | 0.041 | 0.144 | 0.27 | 0.041 | 0.566 | -0.524 |
| cg10161 121 | FASLG | 1 | 29/ NM_0006 39.1 | 0.704 | 0.587 | 0.697 | 0.905 | 0.892 | 0.068 | 0.051 | 0.06 | 0.088 | 0.06 | 0.353 | 0.441 | 0.065 | 0.581 | -0.516 |
| cg00071 250 | FASLG | 1 | 30/ NM_0006 39.1 | 0.659 | 0.498 | 0.602 | 0.871 | 0.838 | 0.075 | 0.061 | 0.069 | 0.139 | 0.051 | 0.391 | 0.4144 6208 | 0.08 | 0.543 | -0.463 |
| cg02473 123 | CD7 | 17 | 31/ NM_0061 | 0.811 | 0.681 | 0.767 | 0.942 | 0.898 | 0.339 | 0.102 | 0.328 | 0.168 | 0.372 | 0.767 | 0.81 | 0.243 | 0.752 | -0.509 |
| cg15518 883 | SIT1 | 9 | 37.6 32/ NM_0144 50.2 | 0.817 | 0.601 | 0.696 | 0.911 | 0.885 | 0.9033 | 0.11 | 0.142 | 0.154 | 0.358 | 0.392 | 0.373 | 0.191 | 0.697 | -0.506 |
| cg18350 391 | IL32 | 16 | 33/ NM_0010 12631.1 | 0.332 | 0.724 | 0.794 | 0.937 | 0.901 | 0.658 | 0.296 | 0.169 | 0.15 | 0.192 | 0.881 | 0.406 | 0.202 | 0.704 | -0.502 |
| cg25226 014 | CXCR 6 | 3 | 34/ NM_0065 64.1 | 0.702 | 0.481 | 0.548 | 0.789 | 0.89 | 0.35 | 0.028 | 0.046 | 0.046 | 0.193 | 0.258 | 0.514 | 0.078 | 0.567 | -0.488 |
| cg13578 652 | UBAS H3A | 21 | 35/ NM_0189 61.2 | 0.564 | 0.444 | 0.475 | 0.537 | 0.703 | 0.169 | 0.027 | 0.04 | 0.035 | 0.05 | 0.602 | 0.673 | 0.038 | 0.521 | -0.483 |
| cg25712 380 | GRAP2 | 22 | 36/ NM_0048 10.2 | 0.633 | 0.602 | 0.639 | 0.824 | 0.83 | 0.506 | 0.097 | 0.093 | 0.089 | 0.1 | 0.179 | 0.310 | 0.095 | 0.565 | -0.470 |
| cg19812 619 | ITGB7 | 12 | 37/ NM_0008 89.1 | 0.819 | 0.651 | 0.785 | 0.874 | 0.897 | 0.645 | 0.3374 | 0.275 | 0.199 | 0.177 | 0.608 | 0.4337 | 0.247 | 0.714 | -0.467 |
| cg02600 394 | TXK | 4 | 38/ NM_0033 28.1 | 0.775 | 0.576 | 0.682 | 0.867 | 0.909 | 0.082 | 0.036 | 0.075 | 0.051 | 0.354 | 0.376 | 0.484 | 0.129 | 0.594 | -0.465 |

## Claims

1. A method for identifying CD3⁺ CD4⁺, and/or CD3⁺ CD8⁺ naive and/or memory T-lymphocytes in a sample derived from a mammal comprising T cells, comprising
a) analysing the methylation status of at least one CpG position in an amplicon comprising amplification with at least one primer pair suitably designed based on oligomers according to any of SEQ ID NO: 2 to 5, and
b) identifying CD3⁺ CD4⁺, and/or CD3⁺ CD8⁺ naive and/or memory T-lymphocytes based on said methylation status, wherein a demethylation of at least one CpG position in said amplicon to at least 90% in said sample is indicative for a CD3⁺ CD4⁺, and/or CD3⁺ CD8⁺ naive and/or memoryT-lymphocyte cell.

2. The method according to claim 1, wherein said at least one CpG position in said sample is demethylated to more than 91% and preferably more than 92% and most preferred more than 95%.

3. The method according to claim 1 or 2, wherein the analysis of the methylation status comprises a method selected from methylation specific enzymatic digests, bisulphite sequencing, analysis selected from promoter methylation, CpG island methylation, MSP, HeavyMethyl, MethyLight, Ms-SNuPE or other methods relying on a detection of amplified DNA.

4. The method according to any of claims 1 to 3, further comprising a methylation analysis of at least one CpG position in the genes for CD4⁺ and/or CD8, in particular CD8 beta, or in the genes for GNGT2, CRTAM, IL2RB, and ZBTB32, or FLJ00060, FLJ38379, PPP6C, CD226, ZBTB7B, and TNFAIP8, or in the genes SLA2, CHRNA3, C16orf24, LCK, FASLG, CD7, SIT1, IL32, CXCR6, UBASH3A, GRAP2, ITGB7 and TXK

5. The method according to any of claims 1 to 4, wherein said method is suitable for routine application, for example on a DNA-chip.

6. The method according to any of claims 1 to 5, wherein said identification comprises a distinction of said T-lymphocytes from all major peripheral blood cell types or non-blood cells.

7. The method according to any of claims 1 to 6, wherein said sample is selected from a mammalian body fluid, including human blood samples, or a tissue, organ or cell type blood sample, a sample of blood lymphocytes or a fraction thereof.

8. The method according to any of claims 1 to 7, further comprising the step of concluding on the immune status of said mammal based on said T-lymphocytes as identified.

9. The method according to any of claims 1 to 8, wherein said mammal suffers from or is likely to suffer from autoimmune diseases, transplant rejections, cancer, and/or allergy.

10. Use of a kit comprising materials for performing the method for identifying CD3⁺ CD4⁺, and/or CD3⁺ CD8⁺ naive and/or memoryT-lymphocytes in a sample according to claim 1, said kit comprising
a) a bisulfite reagent, and
b) materials for performing a methylation analysis for CpG positions in an amplicon comprising amplification with at least one primer pair suitably designed based on oligomers according to any of SEQ NO: 2 to 5
for identifying CD3⁺ CD4⁺, and/or CD3⁺ CD8⁺ naive and/or memoryT-lymphocytes in a mammal.

## Patentansprüche

1. Verfahren zur Identifizierung von CD3⁺ CD4⁺ und/oder CD3⁺ CD8⁺ naïven und/oder Memory- T-Lymphozyten in einer Probe umfassend T-Zellen, die von einem Säugetier abgeleitet ist, umfassend
a) Analysieren des Methylierungsstatus von mindestens einer CpG Position in einem Amplicon, umfassend Amplifikation mit mindestens einem Primerpaar, das basierend auf den Oligomeren gemäß einer der SEQ ID No. 2 bis 5 geeignet aufgebaut ist, und
b) Identifizieren von CD3⁺ CD4⁺ und/oder CD3⁺ CD8⁺ naïven und/oder Memory- T-Lymphozyten basierend auf dem Methylierungsstatus, wobei eine Demethylierung von mindestens einer CpG Position in dem Amplicon zu mindestens 90% in der Probe eine CD3⁺ CD4⁺ und/oder CD3⁺ CD8⁺ naive und/oder Memory- T-Lymphozytenzelle anzeigt.

2. Verfahren nach Anspruch 1, wobei die mindestens eine CpG Position in der Probe zu mehr als 91% demethyliert ist, und bevorzugt zu mehr als 92%, und am meisten bevorzugt zu mehr als 95%.

3. Verfahren nach Anspruch 1 oder 2, wobei die Analyse des Methylierungsstatus ein Verfahren ausgewählt aus Methylierungs-spezifischen enzymatischen Verdaus, Bisulfitsequenzierung, einer Analyse ausgewählt aus Promotormethylierung, CpG Insel-Methylierung, MSP, HeavyMethyl, MethyLight, Ms-SNuPE oder anderen Verfahren, die auf einem Nachweis von amplifizierter DNA beruhen, umfasst.

4. Verfahren nach einem der Ansprüche 1 bis 3, weiter umfassend eine Methylierungsanalyse von mindestens einer CpG Position in den Genen für CD4⁺ und/oder CD8, insbesondere CD8 beta, oder in den Genen für GNGT2, CRTAM, IL2RB und ZBTB32, oder FLJ00060, FLJ38379, PPP6C, CD226, ZBTB7B und TNFAIP8, oder in den Genen SLA2, CHRNA3, C16orf24, LCK, FASLG, CD7, SIT1, IL32, CXCR6, UBASH3A, GRAP2, ITGB7 oder TXK.

5. Verfahren nach einem der Ansprüche 1 bis 4, wobei das Verfahren für die Routineanwendung, zum Beispiel auf einem DNA-Chip, geeignet ist.

6. Verfahren nach einem der Ansprüche 1 bis 5, wobei die Identifizierung eine Unterscheidung der T-Lymphozyten von allen anderen hauptsächlichen peripheren Blutzelltypen oder nicht-Blutzellen umfasst.

7. Verfahren nach einem der Ansprüche 1 bis 6, wobei die Probe ausgewählt ist aus einer Körperflüssigkeit eines Säugetiers, einschließlich menschlicher Blutproben, oder einem Gewebe, Organ oder Zelltyp-Blutprobe, einer Probe von Blutlymphozyten oder einer Fraktion davon.

8. Verfahren nach einem der Ansprüche 1 bis 7, weiter umfassend den Schritt von Schlussfolgern auf den Immunstatus des Säugetiers basierend auf den T-Lymphozyten wie identifiziert.

9. Verfahren nach einem der Ansprüche 1 bis 8, wobei das Säugetier an Autoimmunerkrankungen, Transplantatabstossungen, Krebs und/oder Allergie leidet oder es wahrscheinlich daran leidet.

10. Verwendung eines Kits umfassend Materialien zur Durchführung des Verfahren zur Identifizierung von CD3⁺ CD4⁺ und/oder CD3⁺ CD8⁺ naïven und/oder Memory- T-Lymphozyten in einer Probe nach Anspruch 1, das Kit umfassend
a) ein Bisulfitreagenz, und
b) Materialien zur Durchführung einer Methylierungsanalyse von CpG Positionen in einem Amplicon umfassend Amplifikation mit mindestens einem Primerpaar, das basierend auf den Oligomeren gemäß einer der SEQ ID No. 2 bis 5 geeignet aufgebaut ist
zur Identifizierung von CD3⁺ CD4⁺ und/oder CD3⁺ CD8⁺ naïven und/oder Memory- T-Lymphozyten in einem Säugetier.

## Revendications

1. Procédé permettant d'identifier des lymphocytes T naïfs et/ou mémoire CD3⁺ CD4⁺ et/ou CD3⁺ CD8⁺ dans un échantillon dérivé d'un mammifère comprenant des lymphocytes T, comprenant
a) l'analyse de l'état de méthylation d'au moins une position de CpG dans un amplicon comprenant l'amplification avec au moins une paire d'amorces conçues de façon appropriée en se basant sur des oligomères selon l'une quelconque de SEQ ID NO : 2 à 5, et
b) l'identification des lymphocytes T naïfs et/ou mémoire CD3⁺ CD4⁺ et/ou CD3⁺ CD8⁺ en se basant sur ledit état de méthylation, dans lequel une déméthylation d'au moins une position de CpG dans ledit amplicon jusqu'à au moins 90 % dans ledit échantillon indique une cellule de lymphocyte T naïf et/ou mémoire CD3⁺ CD4⁺ et/ou CD3⁺ CD8⁺.

2. Procédé selon la revendication 1, dans lequel ladite au moins une position de CpG dans ledit échantillon est déméthylée jusqu'à plus de 91 % et de préférence plus de 92 % et de façon préférée entre toutes plus de 95 %.

3. Procédé selon la revendication 1 ou 2, dans lequel l'analyse de l'état de méthylation comprend un procédé choisi parmi des digestions enzymatiques spécifiques de la méthylation, le séquençage au bisulfite, une analyse choisie parmi la méthylation du promoteur, la méthylation d'îlots CpG, la MSP, HeavyMethyl, MethyLight, Ms-SNuPE ou d'autres procédés reposant sur une détection d'ADN amplifié.

4. Procédé selon l'une quelconque des revendications 1 à 3, comprenant en outre une analyse de la méthylation d'au moins une position de CpG dans les gènes pour CD4⁺ et/ou CD8, en particulier CD8 bêta, ou dans les gènes pour GNGT2, CRTAM, IL2RB, et ZBTB32, ou FLJ00060, FLJ38379, PPP6C, CD226, ZBTB7B, et TNFAIP8, ou dans les gènes SLA2, CHRNA3, C16orf24, LCK, FASLG, CD7, SIT1, IL32, CXCR6, UBASH3A, GRAP2, ITGB7 et TXK.

5. Procédé selon l'une quelconque des revendications 1 à 4, ledit procédé étant approprié pour une application en routine, par exemple sur une puce à ADN.

6. Procédé selon l'une quelconque des revendications 1 à 5, dans lequel ladite identification comprend une distinction desdits lymphocytes T de tous les types cellulaires majeurs du sang périphérique ou des cellules non sanguines.

7. Procédé selon l'une quelconque des revendications 1 à 6, dans lequel ledit échantillon est choisi parmi un liquide corporel de mammifère, y compris des échantillons de sang humain, ou un échantillon de sang d'un tissu, d'un organe ou d'un type cellulaire, un échantillon de lymphocytes sanguins ou l'une de leurs fractions.

8. Procédé selon l'une quelconque des revendications 1 à 7, comprenant en outre l'étape de conclusion sur l'état immunitaire dudit mammifère en se basant sur lesdits lymphocytes T tels qu'identifiés.

9. Procédé selon l'une quelconque des revendications 1 à 8, dans lequel ledit mammifère souffre ou est susceptible de souffrir de maladies auto-immunes, de rejets de greffes, de cancer, et/ou d'allergie.

10. Utilisation d'un kit comprenant des matériaux pour effectuer le procédé permettant l'identification des lymphocytes T naïfs et/ou mémoire CD3⁺ CD4⁺ et/ou CD3⁺ CD8⁺ dans un échantillon selon la revendication 1, ledit kit comprenant
a) un réactif de bisulfite, et
b) des matériaux pour effectuer une analyse de la méthylation pour des positions de CpG dans un amplicon comprenant l'amplification avec au moins une paire d'amorces conçues de façon appropriée en se basant sur des oligomères selon l'une quelconque de SEQ ID NO : 2 à 5
pour l'identification des lymphocytes T naïfs et/ou mémoire CD3⁺ CD4⁺ et/ou CD3⁺ CD8⁺ chez un mammifère.


### SEQUENCE LISTING

<110> Epiontis GmbH
<120> Epigenetic markers for the identification of blood sub-cells of type 1
<130> E31285PCT
<150> EP08021838.1-2403
   <151> 2008-12-16
<160> 65
<170> PatentIn version 3.5
<210> 1
   <211> 16501
   <212> DNA
   <213> Homo sapiens
<900> 1
<210> 2
   <211> 50
   <212> DNA
   <213> Homo sapiens
<400> 2
   agctgctgca caggctggct ggctggctgg ctgctaaggg ctgctccacg 50
<210> 3
   <211> 50
   <212> DNA
   <213> Homo sapiens
<400> 3
   cggaaaaaca aaaggcatct gcacctgcag ccctgctgag gcccctgctg 50
<210> 4
   <211> 50
   <212> DNA
   <213> Homo sapiens
<400> 4
   acccaggctg atagttcggt gacctggctt tatctactgg atgagttccg 50
<210> 5
   <211> 50
   <212> DNA
   <213> Homo sapiens
<400> 5
   tggaacatag cacgtttctc tctggcctgg tactggctac ccttctctcg 50
<210> 6
   <211> 435
   <212> DNA
   <213> Homo sapiens
<400> 6
   ttattccacc tattaccttc caaacgcctc tttcataatc tcaaaaaaat aataaaacca 60
   aataccgtat ctcacgccta taatcccaac actttaaaaa actaaaacaa ataaatcaca 120
   aaatcaaaaa ttcgaaacca acctaaccaa caaaataaaa ccccgtctct actaaaaata 180
   caaaaattaa ccgaacttac taacacgcac ctataatctc aactactcaa aaaactaaaa 240
   caaaaaaatc gcttaaaccc cgaaaataaa aattacaata aactaaaata acgccactac 300
   actccaacct aaacaacaaa ataaaactct atctcaaaaa aaaaaaaaaa aaaaaaaata 360
   aaaaaaattc ctaaataaaa acctaaacta aaataacttt ccatttaaaa atccaacccc 420
   aaacatctaa aaatc 435
<210> 7
   <211> 438
   <212> DNA
   <213> Homo sapiens
<400> 7
   ataaacctca ctcccatcaa taatatccct ctcaaatccc ttcccccacc cacctaaaat 60
   aaccttacct tacgaaaaaa aaataaccaa taccaaacca aaaaaaaacg tactatattc 120
   catctcccaa cgaaactcat ccaataaata aaaccaaatc accgaactat caacctaaat 180
   aaaaactacc ctcccctaac taactcacaa ataccgaaaa aaaaaaaata aaaaaaaaac 240
   taaaaaatat ctacttctct actttctacc gttaataata aaaaattttc aaaataaaaa 300
   taaactaaaa ataaaataaa aaaaaaacga tataaattac tctatctcta aacaaaaaac 360
   taacaaaaaa tataaaaaaa ataacctaaa ctttaacgtc cctattaaca atacaaccac 420
   atttacacac aacctaaa 438
<210> 8
   <211> 472
   <212> DNA
   <213> Homo sapiens
<400> 8
   aatccctcct aaattcatta cctacaacaa caacaactaa caacaaataa caactaacta 60
   cgatcctaac aactaataac aaaaacattt atctccctca taaaaaaacg atcccaaaac 120
   catctcccac ccaacatcca ttacgattcc ctatacaaaa taaatctcta aataaaaatc 180
   caacactctc tccctcttct tccccaccac cttcaccctc cttaacgaaa aaacaaaaaa 240
   catctacacc tacaacccta ctaaaacccc tactactcac acttacaaca aaaaataaaa 300
   actctaaatt cttaccttct ctcaaaaacc ccaaccccaa caataataaa taaaaccaat 360
   ctaactacta cacaaactaa ctaactaact aactactaaa aactactcca cgcttttacc 420
   gaaaaacaaa aactaacata aaacaaaaaa aaaacctaac tatcctcatc ct 472
<210> 9
   <211> 50
   <212> DNA
   <213> Homo sapiens
<400> 9
   agtcatctgt tttgcttttt ttccagaagt agtaagtctg ctggcctccg 50
<210> 10
   <211> 50
   <212> DNA
   <213> Homo sapiens
<400> 10
   cgcccagcct cgctgttctt atcttggcag cagattccga atgtcggctg 50
<210> 11
   <211> 50
   <212> DNA
   <213> Homo sapiens
<400> 11
   cgctcatcag gacttcagca ctgcccgtcc atggggacgt ctgcactcac 50
<210> 12
   <211> 50
   <212> DNA
   <213> Homo sapiens
<400> 12
   cgccttgagc tgtacctggc acatctttgt tgctcagcaa agagtggttg 50
<210> 13
   <211> 50
   <212> DNA
   <213> Homo sapiens
<400> 13
   tcttcccatc tgcgtcttgg ttaaggactt ttatcgtatc ctgtttatcg 50
<210> 14
   <211> 50
   <212> DNA
   <213> Homo sapiens
<400> 14
   aagatctgct ggaggccagg cacagacagg tgctttaact caagttatcg 50
<210> 15
   <211> 50
   <212> DNA
   <213> Homo sapiens
<400> 15
   agtaactgca gcaatgagtg cccgggctgt gcttggagta ccagtgctcg 50
<210> 16
   <211> 50
   <212> DNA
   <213> Homo sapiens
<400> 16
   cgccccaagt actaagtggc acagcttggt gagcaaggac atttttattg 50
<210> 17
   <211> 50
   <212> DNA
   <213> Homo sapiens
<400> 17
   cggcaagatg aaacaagctt attaggctct gtcttttaag ggcataccag 50
<210> 18
   <211> 50
   <212> DNA
   <213> Homo sapiens
<400> 18
   cgcaacacag ggctgcctcc cccggtatat gggccccact ccacagaggt 50
<210> 19
   <211> 50
   <212> DNA
   <213> Homo sapiens
<400> 19
   cggcagagca cacagctgca gaagtaaaaa ggattgaaac atttggatcc 50
<210> 20
   <211> 50
   <212> DNA
   <213> Homo sapiens
<400> 20
   acccaggctg atagttcggt gacctggctt tatctactgg atgagttccg 50
<210> 21
   <211> 50
   <212> DNA
   <213> Homo sapiens
<400> 21
   tggaacatag cacgtttctc tctggcctgg tactggctac ccttctctcg 50
<210> 22
   <211> 50
   <212> DNA
   <213> Homo sapiens
<400> 22
   agctgctgca caggctggct ggctggctgg ctgctaaggg ctgctccacg 50
<210> 23
   <211> 50
   <212> DNA
   <213> Homo sapiens
<400> 23
   cggaaaaaca aaaggcatct gcacctgcag ccctgctgag gcccctgctg 50
<210> 24
   <211> 50
   <212> DNA
   <213> Homo sapiens
<400> 24
   agtcatctgt tttgcttttt ttccagaagt agtaagtctg ctggcctccg 50
<210> 25
   <211> 50
   <212> DNA
   <213> Homo sapiens
<400> 25
   cgctgcagtc agactccaaa gtcaggaacg tgagggctac catctctcaa 50
<210> 26
   <211> 50
   <212> DNA
   <213> Homo sapiens
<400> 26
   cgaaggcccc cagctgtggg agcccacgct atttattggt gatcaaagaa 50
<210> 27
   <211> 50
   <212> DNA
   <213> Homo sapiens
<400> 27
   cgcaggcact tacgatcctg ccgtcgccag agcccagatc caccgttttt 50
<210> 28
   <211> 50
   <212> DNA
   <213> Homo sapiens
<400> 28
   gggctcccgg gctgggcagg taaggagcgc tggtattggg ggcgcaggcg 50
<210> 29
   <211> 50
   <212> DNA
   <213> Homo sapiens
<400> 29
   ttccgcccac aattttctga taacagcctt caaggcctca gttgctgtcg 50
<210> 30
   <211> 50
   <212> DNA
   <213> Homo sapiens
<400> 30
   cgggaccctg ttgctgactg ctcaagagga ggcaagctgg atctctctta 50
<210> 31
   <211> 50
   <212> DNA
   <213> Homo sapiens
<400> 31
   taaatgatgt cttggggctg tggcccgagc tgcctcaggt agatcccacg 50
<210> 32
   <211> 50
   <212> DNA
   <213> Homo sapiens
<400> 32
   ggggactgtg ggtcctctta ggggctgtga cgctgctatt tctcatctcg 50
<210> 33
   <211> 50
   <212> DNA
   <213> Homo sapiens
<400> 33
   cgcactcagc aaggcctcct gccctgagag aggctccgcc cactaccccc 50
<210> 34
   <211> 50
   <212> DNA
   <213> Homo sapiens
<400> 34
   ctcatgaagc aaggtctgcc tctacttctg tgatgtggtt catgtttccg 50
<210> 35
   <211> 50
   <212> DNA
   <213> Homo sapiens
<400> 35
   gacctttagc ctcaaactcc cgtggtggaa acagttagga ttggtggacg 50
<210> 36
   <211> 50
   <212> DNA
   <213> Homo sapiens
<400> 36
   gagagacaat acccttccaa aatacacttc aacataaagt tttcttttcg 50
<210> 37
   <211> 50
   <212> DNA
   <213> Homo sapiens
<400> 37
   cggtatacct gccctacttc ggtatagtcc ttggcacatg gcaggcactc 50
<210> 38
   <211> 50
   <212> DNA
   <213> Homo sapiens
<900> 38
   atggtagccc cttctgcggg gagcacacaa cagtcttcag ttcttctgcg 50
<210> 39
   <211> 402
   <212> DNA
   <213> Homo sapiens
<400> 39
   agcctcttta taatctcaaa aaaataataa aaccaaatac cgtatctcac gcctataatc      60
   ccaacacttt aaaaaactaa aacaaataaa tcacaaaatc aaaaattcga aaccaaccta     120
   accaacaaaa taaaaccccg tctctactaa aaatacaaaa attaaccgaa cttactaaca     180
   cgcacctata atctcaacta ctcaaaaaac taaaacaaaa aaatcgctta aaccccgaaa     240
   ataaaaatta caataaacta aaataacgcc actacactcc aacctaaaca acaaaataaa     300
   actctatctc aaaaaaaaaa aaaaaaaaaa aaaaaaaaaa atttctaaaa aaaaacccaa     360
   aaaaaaaaaa cttttccttt aaaaaacccc ccccaaaaat tt                        402
<210> 40
   <211> 394
   <212> DNA
   <213> Homo sapiens
<400> 40
   agcctcttta taatctcaaa aaaataataa aaccaaatac cgtatctcac gcctataatc      60
   ccaacacttt aaaaaactaa aacaaataaa tcacaaaatc aaaaattcga aaccaaccta     120
   accaacaaaa taaaaccccg tctctactaa aaatacaaaa attaaccgaa cttactaaca     180
   cgcacctata atctcaacta ctcaaaaaac taaaacaaaa aaatcgctta aaccccgaaa     240
   ataaaaatta caataaacta aaataacgcc actacactcc aacctaaaca acaaaataaa     300
   actctatctc aaaaaaaaaa aaaaaaaaaa aaaaaaaaaa atttccaaaa aaaaacccaa     360
   aaaaaaaaaa ctttcccttt aaaaaacccc cccc                                 394
<210> 41
   <211> 435
   <212> DNA
   <213> Homo sapiens
<400> 41
   ttattccacc tattaccttc caaacgcctc tttcataatc tcaaaaaaat aataaaacca 60
   aataccgtat ctcacgccta taatcccaac actttaaaaa actaaaacaa ataaatcaca 120
   aaatcaaaaa ttcgaaacca acctaaccaa caaaataaaa ccccgtctct actaaaaata 180
   caaaaattaa ccgaacttac taacacgcac ctataatctc aactactcaa aaaactaaaa 240
   caaaaaaatc gcttaaaccc cgaaaataaa aattacaata aactaaaata acgccactac 300
   actccaacct aaacaacaaa ataaaactct atctcaaaaa aaaaaaaaaa aaaaaaaata 360
   aaaaaaattc ctaaataaaa acctaaacta aaataacttt ccatttaaaa atccaacccc 420
   aaacatctaa aaatc 435
<210> 42
   <211> 399
   <212> DNA
   <213> Homo sapiens
<400> 42
   cgcctcttta taatctcaaa aaaataataa aaccaaatac cgtatctcac gcctataatc 60
   ccaacacttt aaaaaactaa aacaaataaa tcacaaaatc aaaaattcga aaccaaccta 120
   accaacaaaa taaaaccccg tctctactaa aaatacaaaa attaaccgaa cttactaaca 180
   cgcacctata atctcaacta ctcaaaaaac taaaacaaaa aaatcgctta aaccccgaaa 240
   ataaaaatta caataaacta aaataacgcc actacactcc aacctaaaca acaaaataaa 300
   actctatctc aaaaaaaaaa aaaaaaaaaa aaaaaaaaaa atttccaaaa aaaaacccaa 360
   aaaaaaaaaa ctttcccttt aaaaaacccc ccccaaaaa 399
<210> 43
   <211> 394
   <212> DNA
   <213> Homo sapiens
<400> 43
   tacctcttta taaatctcaa aaaaataata aaaccaaata ccatatctca cacctataat 60
   cccaacactt taaaaaacta aaacaaataa atcacaaaat caaaaattca aaaccaacct 120
   aaccaacaaa ataaaacccc atctctacta aaaatacaaa aattaaccaa acttactaac 180
   acacacctat aatctcaact actcaaaaaa ctaaaacaaa aaaatcactt aaaccccaaa 240
   aataaaaatt acaataaact aaaataacac cactacactc caacctaaac aacaaaataa 300
   aactctatct caaaaaaaaa aaaaaaaaaa aaaaaaaaaa aatttccaaa aaaaaaccca 360
   aaaaaaaaaa actttccctt taaaaacccc cccc 394
<210> 44
   <211> 396
   <212> DNA
   <213> Homo sapiens
<400> 44
   gacctcttta taatctcaaa aaaataataa aaccaaatac catatctcac acctataatc 60
   ccaacacttt aaaaaactaa aacaaataaa tcacaaaatc aaaaattcaa aaccaaccta 120
   accaacaaaa taaaacccca tctctactaa aaatacaaaa attaaccaaa cttactaaca 180
   cacacctata atctcaacta ctcaaaaaac taaaacaaaa aaatcactta aaccccaaaa 240
   ataaaaatta caataaacta aaataacacc actacactcc aacctaaaca acaaaataaa 300
   actctatctc aaaaaaaaaa aaaaaaaaaa aaaaaaaaaa attcccaaaa aaaaacccaa 360
   aaaaaaaaaa ctttcccttt aaaaaccccc ccccaa 396
<210> 45
   <211> 387
   <212> DNA
   <213> Homo sapiens
<400> 45
   cgcctcttta tatctaaaaa aatataaacc aataccgtat ctcacaccta taatcccaac 60
   acttaaaaaa ctaaaacaaa taaatcacaa aatcaaaaat tcagaaacca acctaaccaa 120
   caaaataaaa ccccgtctct actaaaaata caaaaattaa ccaaacttac taacacacac 180
   ctataatctc aactactcaa aaaactaaaa caaaaaaatc acttaaaccc caaaaataaa 240
   aattacaata aactaaaata acaccactac actccaacct aaacaacaaa ataaaactct 300
   atctcaaaaa aaaaaaaaaa aaaaaaaaaa aaaaaatttc caaaaaaaaa cccaaaaaaa 360
   aaaaactttc cctttaaaaa acccccc 387
<210> 46
   <211> 391
   <212> DNA
   <213> Homo sapiens
<400> 46
   atgattgtta atagggacgt taaagtttag gttatttttt ttatattttt tgttagtttt 60
   ttgtttagag atagagtaat ttatatcgtt ttttttttat tttattttta gtttattttt 120
   attttgaaaa ttttttatta ttaacggtag aaagtagaga agtagatatt ttttagtttt 180
   ttttttattt tttttttttc ggtttttggg atttatttag gggggggtgt tttttttttg 240
   ggttgatagt tcggggattt ggttttattt atgggaggat ttccgtgggg aaaggaaaaa 300
   aaaacctttt tttttgggtt gggtttgggt tatttttttt ccgaagggaa aggtttttta 360
   aggggggggg ggaaagggtt ttaaaaggaa a 391
<210> 47
   <211> 328
   <212> DNA
   <213> Homo sapiens
<400> 47
   ctatttgatg ggagaagtga ggtgttaata agtttaggtt atttttttta tattttttgt 60
   tagttttttg tttagagata gagtaattta tattgttttt tttttatttt atttttagtt 120
   tatttttatt ttgaaaattt tttattatta atggtagaaa gtagagaagt agatattttt 180
   tagttttttt tttatttttt ttttttgggt ttttgggatt tatttggggg ggggtgtttt 240
   ttttttgggt tgatatttgg gggattgggt ttttttaatg ggaggatttt tgtggggaaa 300
   ggaaataaaa tttttttttt ttgggttg 328
<210> 48
   <211> 388
   <212> DNA
   <213> Homo sapiens
<400> 48
   ctatttgatg ggagaagtga ggttgttaat aagtttaggt tatttttttt atattttttg 60
   ttagtttttt gtttagagat agagtaattt atattgtttt ttttttattt tatttttagt 120
   ttatttttat tttgaaaatt ttttattatt aatggtagaa agtagagaag tagatatttt 180
   ttagtttttt ttttattttt tttttttggg attttggaat taattaaggg gggggaattt 240
   tttttttagg ttgaaatttg ggggattggg ttttattttt gggaggattt ttgtggggaa 300
   aggaaaaaaa tttttttttt tttgggttgg gtttgggttt tttttttttg gaagggaagg 360
   gtttttttgg gggggggggg aaggggtt 388
<210> 49
   <211> 401
   <212> DNA
   <213> Homo sapiens
<400> 49
   cattgatggg aagtagagga ttgataaagt ttaggttatt ttttttatat tttttgttag 60
   ttttttgttt agagatagag taatttatat tgtttttttt ttattttatt tttagtttat 120
   ttttattttg aaaatttttt attattaatg gtagaaagta gagaagtaga tattttttag 180
   tttttttttt attttttttt tttgggtttt tggaattaat ttgggggggg gaattttttt 240
   tttaggtgga aatttggggg attgggtttt atttatggga gaatttttgt ggggaaagga 300
   aataaatttt tttttttttg ggttgggttt gggttttttt tttttgggag ggaagggttt 360
   ttttgggggg gggggggagg ggtttaaagg aattttttgg g 401
<210> 50
   <211> 387
   <212> DNA
   <213> Homo sapiens
<400> 50
   attggtatgt gttaagtatg ggatgtgtaa agtttaggtt atttttttta tattttttgt 60
   tagttttttg tttagagata gagtaattta tattgttttt tttttatttt atttttagtt 120
   tatttttatt ttgaaaattt tttattatta atggtagaaa gtagagaagt agatattttt 180
   tagttttttt tttatttttt ttttttgggt ttttggaatt aattaagggg ggggaatttt 240
   tttttaaggt ggaaagttgg gggattgggt tttatttatg ggaggatttt tgtggggaaa 300
   ggaaaaaaat tttttttttt ttgggttggg tttgggtttt ttttttttgg aagggaaggg 360
   tttttttggg ggggggggga aggggtt 387
<210> 51
   <211> 420
   <212> DNA
   <213> Homo sapiens
<400> 51
   attgttaata gggacgttaa agtttaggtt atttttttta tattttttgt tagttttttg        60
   tttagagata gagtaattta tatcgttttt tttttatttt atttttagtt tatttttatt       120
   ttgaaaattt tttattatta acggtagaaa gtagagaagt agatattttt tagttttttt      180
   tttatttttt tttttccgga atttgtgagt tagtaggggg agggtagttt ttatttaggt       240
   tgatagttcg gtgatttggt ttaatttatt gaatgagttt cgttggggag atgaaatata     300
   gtacggtttt tttttggttt gggtatgggt attttttttt cggaaagggt aaagggtatt     360
   ttaggtgggg tgggggaaag ggattttgga gagggattat attgatgggg agtgaaggtt     420
<210> 52
   <211> 416
   <212> DNA
   <213> Homo sapiens
<400> 52
   tatccctctc aaatcccttc ccccacccac ctaaaataac cttaccttac gaaaaaaaaa      60
   taaccaatac caaaccaaaa aaaaacgtac tatattccat ctcccaacga aactcatcca     120
   ataaataaaa ccaaatcacc gaactatcaa cctaaataaa aactaccctc ccctaactaa     180
   ctcacaaata ccgaaaaaaa aaaaataaaa aaaaacctaa aaaatttttc cttctttcct      240
   ttctcccgtt aaaaaaaaaa aattttcaaa aaaaaaataa cctaaaaaaa aaaaaaaaaa     300
   aaaccgtttt aatttccttt ttttttaacc aaaaaattaa caaaaaattt aaaaaaaata     360
   cccaaaattt taccgcccct ttaaacaatc cacccacttt tccccacacc taaaaa         416
<210> 53
   <211> 416
   <212> DNA
   <213> Homo sapiens
<400> 53
   tatccctctc aaatcccttc ccccacccac ctaaaataac cttaccttac gaaaaaaaaa      60
   taaccaatac caaaccaaaa aaaaacgtac tatattccat ctcccaacga aactcatcca     120
   ataaataaaa ccaaatcacc gaactatcaa cctaaataaa aactaccctc ccctaactaa     180
   ctcacaaata ccgaaaaaaa aaaaataaaa aaaaaactaa aaaatttttc cttctttcct      240
   ttctcccgtt aaaaaaaaaa aattttcaaa aaaaaaataa cctaaaaata aaaaaaaaaa     300
   aaaccgtttt aatttccttt ttttttaacc aaaaacttaa caaaaaattt aaaaaaaaaa 360
   ccctaacttt taccgcccct ttaaacaatc cacccccttt tccccccacc ctaaaa 416
<210> 54
   <211> 418
   <212> DNA
   <213> Homo sapiens
<400> 54
   gtatccctct caaatccctt cccccaccca cctaaaataa ccttacctta cgaaaaaaaa 60
   ataaccaata ccaaaccaaa aaaaaacgta ctatattcca tctcccaacg aaactcatcc 120
   aataaataaa accaaatcac cgaactatca acctaaataa aaactaccct cccctaacta 180
   actcacaaat accgaaaaaa aaaaaataaa aaaaaaacta aaaaattttt tttttttttt 240
   tttttcccgt taataaaaaa aatttttaaa aataaaaata acctaaaaat aaaataaaaa 300
   aaaaccgttt taatttcctt tttttttaac caaaaactta ccaaaaaatt taaaaaaaat 360
   accctaactt ttaccgcccc tttaaaaaaa ccaccccctt ttccccccac ccaaaaaa 418
<210> 55
   <211> 419
   <212> DNA
   <213> Homo sapiens
<400> 55
   gccatcttac ccacaaccct taaccccacc cacctaaaat aaccttacct tacaaaaaaa 60
   aaataaccaa taccaaacca aaaaaaaaca tactatattc catctcccaa caaaactcat 120
   ccaataaata aaaccaaatc accaaactat caacctaaat aaaaactacc ctcccctaac 180
   taactcacaa atcccaaaaa aaaaaaaata aaaaaaaacc taaaaatttt ttttttcttt 240
   cttttccccc tttaaaaaaa aaaattttta aaaaaaaaaa aaacctaaaa aaaaaaaaaa 300
   aaaaaaccat tttaatttct ttttttttta accaaaaact tacaaaaaat tttaaaaaaa 360
   aaccccaaac tttaaccccc cttttaaaaa aaccccccct ttttcccccc ccccaaaaa 419
<210> 56
   <211> 418
   <212> DNA
   <213> Homo sapiens
<400> 56
   gccatcttac ccacaaccct aaccccaccc acctaaaata accttacctt acaaaaaaaa      60
   aataaccaat accaaaccaa aaaaaaacat actatattcc atctcccaac aaaactcatc     120
   caataaataa aaccaaatca ccaaactatc aacctaaata aaaactaccc tcccctaact     180
   aactcacaaa tcccaaaaaa aaaaaaataa aaaaaaacct aaaaattttc tctttctctc     240
   ttttccccct taaaaaaaaa aaatttttaa aaaaaaaaaa aacctaaaaa aaaaaaaaaa    300
   aaaaaccatt ttaatttctt ttttttttaa caaaaaactt acaaaaaatt ttaaaaaaaa     360
   aacccaaact tttacccccc ttttaaaaaa acccccccct tttccccccc cccaaaaa      418
<210> 57
   <211> 419
   <212> DNA
   <213> Homo sapiens
<400> 57
   tatatcctac tcaacatccc tatcccccac ccacctaaaa taaccttacc ttacaaaaaa      60
   aaaataacca ataccaaacc aaaaaaaaac atactatatt ccatctccca acaaaactca     120
   tccaataaat aaaaccaaat caccaaacta tcaacctaaa taaaaactac cctcccctaa     180
   ctaactcaca aatcccaaaa aaaaaaaaaa aaaaaaaaac ctaaaaattt tttctttttt     240
   tctttttccc cttaaaaaaa aaaaattttt aaaaaaaaaa aaaacctaaa aaaaaaaaaa     300
   aaaaaaacct ttttaatttc tcctttcttt aaccaaaaac ttaccaaaaa ttttaaaaaa     360
   aatccccaaa ttttaacccc ccttttaaaa aaaccacccc tttttccccc cccccaaaa      419
<210> 58
   <211> 416
   <212> DNA
   <213> Homo sapiens
<400> 58
   catccctctc taatcccttc ccccacccac ctaaaataac cttaccttac aaaaaaaaaa      60
   taaccaatac caaaccaaaa aaaaacatac tatattccat ctcccaacaa aactcatcca     120
   ataaataaaa ccaaatcacc aaactatcaa cctaaataaa aactaccctc ccctaactaa     180
   ctcacaaatc ccaaaaaaaa aaaaataaaa aaaaacctaa aaaatttttc tttttttctt     240
   tttcccctta aaaaaaaaaa atttttaaaa aaaaaattaa cttaaaataa aaaaaaaaaa     300
   aaaccatttt aatttttttt ttttttaaca aaaaacttac caaaaatttt aaaaaaaaac     360
   cccaaacttt tacccccctt ttaaaaaacc cccccctttt tccccccccc caaaaa        416
<210> 59
   <211> 438
   <212> DNA
   <213> Homo sapiens
<400> 59
   ctataacaac aacaactaac aacaaataac aactaactac aatcctaaca actaataaca      60
   aaaacattta tctccctcat aaaaaaacaa tcccaaaacc atctcccacc caacatccat     120
   tacaattccc tatacaaaat aaatctctaa ataaaaatcc aacactctct ccctcttctt     180
   ccccaccacc ttcaccctcc ttaacaaaaa aacaaaaaac atctacacct acaaccctac     240
   taaaacccct actactcaca cttacaacaa aaaataaaaa ctctaaattc ttaccttctc     300
   tcaaaaaccc caaccccaac aataataaat aaaaccaatc taactactac acaaactaac     360
   taactaacta actacaaaaa actactcctt acttttacca aaaaacacaa actaacataa     420
   aacaaaaaaa aaacctat                                                   438
<210> 60
   <211> 442
   <212> DNA
   <213> Homo sapiens
<400> 60
   ctataacaac aacaactaac aacaaataac aactaactac aatcctaaca actaataaca      60
   aaaacattta tctccctcat aaaaaaacaa tcccaaaacc atctcccacc caacatccat     120
   tacaattccc tatacaaaat aaatctctaa ataaaaatcc aacactctct ccctcttctt     180
   ccccaccacc ttcaccctcc ttaacaaaaa aacaaaaaac atctacacct acaaccctac     240
   taaaacccct actactcaca cttacaacaa aaaataaaaa ctctaaattc ttaccttctc     300
   tcaaaaaccc caaccccaac aataataaat aaaaccaatc taactactac acaaactaac     360
   taactaacta actacaaaaa actactcctt acttttacca aaaaacacaa actaacataa     420
   aacaaaaaaa aaacctatct tt                                              442
<210> 61
   <211> 445
   <212> DNA
   <213> Homo sapiens
<400> 61
   ctataacaac aacaactaac aacaaataac aactaactac aatcctaaca actaataaca 60
   aaaacattta tctccctcat aaaaaaacaa tcccaaaacc atctcccacc caacatccat 120
   tacaattccc tatacaaaat aaatctctaa ataaaaatcc aacactctct ccctcttctt 180
   ccccaccacc ttcaccctcc ttaacaaaaa aacaaaaaac atctacacct acaaccctac 240
   taaaacccct actactcaca cttacaacaa aaaataaaaa ctctaaattc ttaccttctc 300
   tcaaaaaccc caaccccaac aataataaat aaaaccaatc taactactac acaaactaac 360
   taactaacta actacaaaaa actactcctt acttttacca aaaaacacaa actaacataa 420
   aacaaaaaaa aaacctatct ttcct 445
<210> 62
   <211> 437
   <212> DNA
   <213> Homo sapiens
<400> 62
   gtataacaac aacaactaac aacaaataac aactaactac aatcctaaca actaataaca 60
   aaaacattta tctccctcat aaaaaaacaa tcccaaaacc atctcccacc caacatccat 120
   tacaattccc tatacaaaat aaatctctaa ataaaaatcc aacactctct ccctcttctt 180
   ccccaccacc ttcaccctcc ttaacaaaaa aacaaaaaac atctacacct acaaccctac 240
   taaaacccct actactcaca cttacaacaa aaaataaaaa ctctaaattc ttaccttctc 300
   tcaaaaaccc caaccccaac aataataaat aaaaccaatc taactactac acaaactaac 360
   taactaacta actacaaaaa actactcctt acttttacca aaaaacacaa actaacataa 420
   aacaaaaaaa aaaccta 437
<210> 63
   <211> 450
   <212> DNA
   <213> Homo sapiens
<400> 63
   tataacaaca acaactaaca acaaataaca actaactacg atcctaacaa ctaataacaa 60
   aaacatttat ctccctcata aaaaaacgat cccaaaacca tctcccaccc aacatccatt 120
   acgattccct atacaaaata aatctctaaa taaaaatcca acactctctc cctcttcttc 180
   cccaccacct tcaccctcct taacgaaaaa acaaaaaaca tctacaccta caaccctact 240
   aaaaccccta ctactcacac ttacaacaaa aaataaaaac tctaaattct taccttctct 300
   caaaaacccc aaccccaaca ataataaata aaaccaatct aactactaca caaactaact 360
   aactaactaa ctactaaaaa ctactgcacg cttttaccga aaaacacaaa ctaacataaa 420
   acaaaaaaaa aacctatctt tccccaacct 450
<210> 64
   <211> 446
   <212> DNA
   <213> Homo sapiens
<400> 64
   tataacaaca acaactaaca acaaataaca actaactacg atcctaacaa ctaataacaa 60
   aaacatttat ctccctcata aaaaaacgat cccaaaacca tctcccaccc aacatccatt 120
   acgattccct atacaaaata aatctctaaa taaaaatcca acactctctc cctcttcttc 180
   cccaccacct tcaccctcct taacgaaaaa acaaaaaaca tctacaccta caaccctact 240
   aaaaccccta ctactcacac ttacaacaaa aaataaaaac tctaaattct taccttctct 300
   caaaaacccc aaccccaaca ataataaata aaaccaatct aactactaca caaactaact 360
   aactaactaa ctactaaaaa ctactgcacg ctttgaccga aaaacaaaaa ctaacataaa 420
   acaaaaaaaa aacctatctt tcccct 446
<210> 65
   <211> 448
   <212> DNA
   <213> Homo sapiens
<400> 65
   gataacaaca acaactaaca acaaataaca actaactacg atcctaacaa ctaataacaa      60
   aaacatttat ctccctcata aaaaaacgat cccaaaacca tctcccaccc aacatccatt     120
   acgattccct atacaaaata aatctctaaa taaaaatcca acactctctc cctcttcttc     180
   cccaccacct tcaccctcct taacgaaaaa acaaaaaaca tctacaccta caaccctact     240
   aaaaccccta ctactcacac ttacaacaaa aaataaaaac tctaaattct taccttctct     300
   caaaaacccc aaccccaaca ataataaata aaaccaatct aactactaca caaactaact     360
   aactaactac taacaactac ttactgcttg ctccgaccaa caaacactaa cttaaaacaa     420
   aaaaaaaaca aacctatctt tctcccaa                                        448